(19) **Europäisches Patentamt**
**European Patent Office**
**Office européen des brevets**

(11) **EP 4 005 468 B1**

(12) **EUROPEAN PATENT SPECIFICATION**

(45) Date of publication and mention
of the grant of the patent:
**30.08.2023 Bulletin 2023/35**

(51) International Patent Classification (IPC):
**A61B 5/00** (2006.01)

(21) Application number: **21210224.8**

(52) Cooperative Patent Classification (CPC):
**A61B 5/40; A61B 5/7253**

(22) Date of filing: **24.11.2021**

(54) **AUTONOMIC NERVE INDEX CALCULATION SYSTEM, AUTONOMIC NERVE INDEX CALCULATION METHOD, AND AUTONOMIC NERVE INDEX CALCULATION PROGRAM**

SYSTEM ZUR BERECHNUNG DES AUTONOMEN NERVENINDEX, VERFAHREN ZUR BERECHNUNG DES AUTONOMEN NERVENINDEX UND PROGRAMM ZUR BERECHNUNG DES AUTONOMEN NERVENINDEX

SYSTÈME DE CALCUL D'INDICE DE NERF AUTONOME, PROCÉDÉ DE CALCUL D'INDICE DE NERF AUTONOME ET PROGRAMME DE CALCUL D'INDICE DE NERF AUTONOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.11.2020 JP 2020195961**

(43) Date of publication of application:
**01.06.2022 Bulletin 2022/22**

(73) Proprietor: **TOYOTA JIDOSHA KABUSHIKI KAISHA**
**Toyota-shi, Aichi-ken, 471-8571 (JP)**

(72) Inventors:
 • **HAYASHI, Keiji**
 **Toyota-shi, 471-8571 (JP)**

 • **YAMADA, Hitoshi**
 **Toyota-shi, 471-8571 (JP)**
 • **YAMAGUCHI, Yuhei**
 **Toyota-shi, 471-8571 (JP)**
 • **ARAI, Kyosuke**
 **Toyota-shi, 471-8571 (JP)**

(74) Representative: **D Young & Co LLP**
**120 Holborn**
**London EC1N 2DY (GB)**

(56) References cited:
**JP-A- 2000 296 118    JP-B2- 5 408 751**

**EP 4 005 468 B1**

**Description**

BACKGROUND

The present disclosure relates to an autonomic nerve index calculation system, an autonomic nerve index calculation method, and an autonomic nerve index calculation program for calculating an autonomic nerve index of a living body.

In related art, various techniques for calculating an autonomic nerve index of a living body have been proposed. As an example of such a technique, an autonomic nerve function measuring apparatus disclosed in Japanese Patent No. 5408751 calculates power spectral density of an amplitude variation of a pulse wave using the Fourier transformation, and calculates a ratio of a low frequency component to a high frequency component of the spectral density as an index of a autonomic nerve function.

JP2000296118A discloses a method and device for analyzing living body signal.

SUMMARY

However, the autonomic nerve function measuring apparatus disclosed in Japanese Patent No. 5408751 has a problem that it cannot calculate a definite index, although the automatic nervous function measuring apparatus processes physiological signals such as a heart rate and a pulse wave, and indexes spectral intensity and phase information of a low frequency component (0.04 Hz to 0.15 Hz: blood pressure fluctuation component) and a high frequency component (0.15 Hz to 0.4 Hz: respiratory fluctuation component) to present the indices as indices of the autonomic nerves, because indices change every moment.

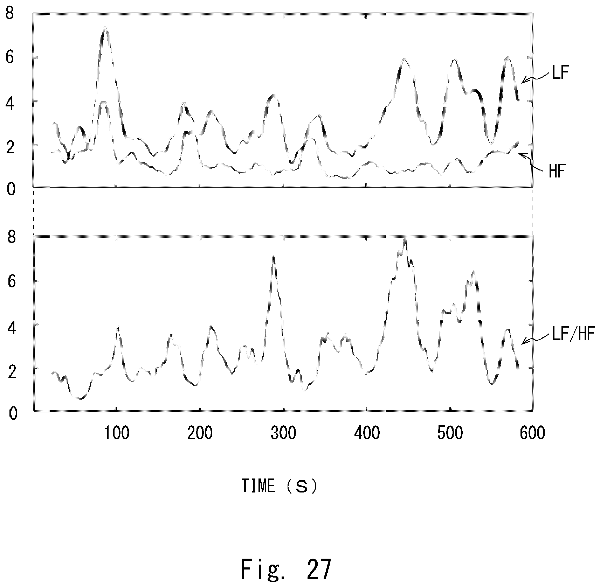

Fig. 27 shows the result of measuring a resting state by the LF/HF evaluation method according to related art. It is said that the lower the LF/HF ratio, the more predominant the parasympathetic nerve system becomes. However, it can be seen that the LF and HF change from moment to moment and the LF/HF changes drastically. One possible reason for this is that it is difficult to perceive physiological indices as scalar quantities, because the variance of the physiological indices increases in a relaxed state and decreases in a tensed state.

An object of the present disclosure is to provide an autonomic nerve index calculation system, an autonomic nerve index calculation method, and an autonomic nerve index calculation program capable of calculating an autonomic nerve index, which is not an autonomic nerve index according to the related art that changes from moment to moment but is substantially a constant numerical value at all times when the state of a body does not change.

An example aspect of the present disclosure is an autonomic nerve index calculation system for calculating an autonomic nerve index of a living body. The autonomic nerve index calculation system includes:

> a pulse wave waveform generation unit configured to generate pulse wave waveform data using at least one pulse wave signal of the living body;
> a band-pass filter configured to filter the generated pulse wave waveform data in at least one predetermined frequency band;
> a complex number conversion unit configured to convert the filtered pulse wave waveform data into a complex number and calculate pulse wave complex waveform data of the at least one predetermined frequency band; and
> an index calculation unit configured to calculate the autonomic nerve index of the living body in the at least one predetermined frequency band based on the calculated pulse wave complex waveform data.

Another example aspect of the present disclosure is an autonomic nerve index calculation system for calculating an autonomic nerve index of a living body. The autonomic nerve index calculation system includes:

> a heartbeat interval waveform generation unit configured to generate heartbeat interval waveform data using at least one pulse wave signal of the living body;
> a band-pass filter configured to filter the generated heartbeat interval waveform data in at least one predetermined frequency band;
> a complex number conversion unit configured to convert the filtered heartbeat interval waveform data into a complex number and calculate heartbeat interval complex waveform data of the at least one predetermined frequency band; and
> an index calculation unit configured to calculate the autonomic nerve index of the living body in the at least one predetermined frequency band based on the calculated heartbeat interval complex waveform data.

Another example aspect of the present disclosure is an autonomic nerve index calculation system for calculating an autonomic nerve index of a living body. The autonomic nerve index calculation system includes:

a pulse wave waveform generation unit configured to generate pulse wave waveform data using at least one pulse wave signal of the living body;

a heartbeat interval waveform generation unit configured to generate heartbeat interval waveform data using at least one pulse wave signal of the living body;

a band-pass filter configured to filter the generated pulse wave waveform data and the heartbeat interval waveform data in at least one predetermined frequency band;

a complex number conversion unit configured to convert the filtered pulse wave data and the filtered heartbeat interval waveform data into complex numbers and calculate pulse wave complex waveform data and heartbeat interval complex waveform data of the at least one predetermined frequency band; and

an index calculation unit configured to calculate the autonomic nerve index of the living body in the at least one predetermined frequency band based on the calculated pulse wave complex waveform data and the calculated heartbeat interval complex waveform data.

Another example aspect of the present disclosure is an autonomic nerve index calculation method for calculating an autonomic nerve index of a living body. The autonomic nerve index calculation method includes:

generating pulse wave waveform data using at least one pulse wave signal of the living body;
filtering the generated pulse wave waveform data in at least one predetermined frequency band;
converting the filtered pulse wave waveform data into a complex number and calculate pulse wave complex waveform data of the at least one frequency band; and
calculating the autonomic nerve index of the living body in the at least one frequency band based on the calculated pulse wave complex waveform data.

Another example aspect of the present disclosure is an autonomic nerve index calculation method for calculating an autonomic nerve index of a living body. The autonomic nerve index calculation method includes:

generating heartbeat interval waveform data using at least one pulse wave signal of the living body;
filtering the generated heartbeat interval waveform data in at least one predetermined frequency band;
converting the filtered heartbeat interval waveform data into a complex number and calculate heartbeat interval complex waveform data of the at least one frequency band; and
calculating the autonomic nerve index of the living body in the at least one frequency band based on the calculated heartbeat interval complex waveform data.

Another example aspect of the present disclosure is an autonomic nerve index calculation method for calculating an autonomic nerve index of a living body. The autonomic nerve index calculation method includes:

generating pulse wave waveform data using at least one pulse wave signal of the living body;
generating heartbeat interval waveform data using at least one pulse wave signal of the living body;
filtering the generated pulse wave waveform data and the heartbeat interval waveform data in at least one predetermined frequency band;
converting the filtered pulse wave data and the filtered heartbeat interval waveform data into complex numbers and calculate pulse wave complex waveform data and heartbeat interval complex waveform data of the at least one frequency band; and
calculating the autonomic nerve index of the living body in the at least one frequency band based on the calculated pulse wave complex waveform data and the calculated heartbeat interval complex waveform data.

Another example aspect of the present disclosure is an autonomic nerve index calculation program for calculating an autonomic nerve index of a living body for causing an information processing apparatus to execute:

generating pulse wave waveform data using at least one pulse wave signal of the living body;
filtering the generated pulse wave waveform data in at least one predetermined frequency band;
converting the filtered pulse wave waveform data into a complex number and calculate pulse wave complex waveform data of the at least one frequency band; and
calculating the autonomic nerve index of the living body in the at least one frequency band based on the calculated pulse wave complex waveform data.

Another example aspect of the present disclosure is an autonomic nerve index calculation program for calculating an autonomic nerve index of a living body for causing an information processing apparatus to execute:

generating heartbeat interval waveform data using at least one pulse wave signal of the living body;
filtering the generated heartbeat interval waveform data in at least one predetermined frequency band;
converting the filtered heartbeat interval waveform data into a complex number and calculate heartbeat interval complex waveform data of the at least one frequency band; and
calculating the autonomic nerve index of the living body in the at least one frequency band based on the calculated heartbeat interval complex waveform data.

Another example aspect of the present disclosure is an autonomic nerve index calculation program for calculating an autonomic nerve index of a living body for causing an information processing apparatus to execute:

generating pulse wave waveform data using at least one pulse wave signal of the living body;
generating heartbeat interval waveform data using at least one pulse wave signal of the living body;
filtering the generated pulse wave waveform data and the heartbeat interval waveform data in at least one predetermined frequency band;
converting the filtered pulse wave data and the filtered heartbeat interval waveform data into complex numbers and calculate pulse wave complex waveform data and heartbeat interval complex waveform data of the at least one frequency band; and
calculating the autonomic nerve index of the living body in the at least one frequency band based on the calculated pulse wave complex waveform data and the calculated heartbeat interval complex waveform data.

Pulse wave waveform data B(t) and pulse wave interval waveform data R(t) are divided into at least four bands of at least h: 0.15 Hz to 0.4 Hz, l: 0.04 Hz to 0.15 Hz, v: 0.015 Hz to 0.04 Hz, and w: 0.004 Hz to 0.015 Hz, waveforms are extracted, and each of the waveforms is subjected to Hilbert transformation to generate a complex waveform function.
[Equation 1]

$$\text{pulse wave waveform}: \quad \psi k(t) = exp(\, ak(t) + i\omega k(t)t\,) \qquad (1)$$

[Equation 2]

$$\text{heartbeat interval waveform}: \quad \Psi_{k(t)} = exp(\, Ak(t) + i\Omega k(t)t\,) \qquad (2)$$

[Equation 3]

$$\text{phase difference}: \quad \theta k(t) = \Omega k(t)t - \omega k(t)t \qquad (3)$$

Here, k: h, l, v, w, ..... Further, i is a complex number, and t is time.
When the pulse wave is measured for at least 20 minutes to 2 hours under the condition that the state of the body does not change, and Equations (1), (2) and (3) are calculated, the time series data of $ak$, $Ak$, $\omega k$, $Qk$, and $\theta k$ are distributed in a normal distribution. When the physiological function allocated to each band is tensed, a variance of the physiological function decreases, while when the physiological function is relaxed, the variance of the physiological function increases.
In related art, a scalar value has been used as an autonomic nerve evaluation index using an HF(h) band in which respiratory fluctuation appears and an LF(1) band in which blood pressure fluctuation appears. However, even for a VLF band (0.004 Hz to 0.04 Hz), which has recently attracted attention as an index of the autonomic nerve system, the band is divided into two bands of v: 0.015 Hz to 0.04 Hz and w: 0.004 Hz to 0.015 Hz to calculate Equations (1) and (2), and the distribution coefficient is obtained.
Since a biological response to various environmental stimuli appears in distribution coefficients of $ak$ and $\omega k$, states of tension and relaxation of a physiological center related to each band can be quantified according to magnitudes of the distribution coefficients, and various states of the autonomic nerve can be described.
Furthermore, even for a low-frequency ULF band (0.0004 Hz to 0.004 Hz) or a cycle longer than that of the ULF band, a very long measurement time (several hours to several months) is required. However, by performing the data processing similar to the processing described above on the measured pulse wave data to calculate $ak$, $\omega k$, etc., it is possible to describe a biological condition having a property different from that described above.
According to the present disclosure, it is possible to provide an autonomic nerve index calculation system, an autonomic nerve index calculation method, and an autonomic nerve index calculation program capable of calculating an autonomic nerve index which is is substantially a constant numerical value at all times when a state of a body does not change.

The above and other objects, features and advantages of the present disclosure will become more fully understood from the detailed description given hereinbelow and the accompanying drawings which are given by way of illustration only, and thus are not to be considered as limiting the present disclosure. Particular aspects of the invention are set out in the appended claims.

BRIEF DESCRIPTION OF DRAWINGS

Fig. 1 shows definitions of a pulse wave waveform and a pulse interval;
Fig. 2 shows a definition of a pulse interval waveform;
Fig. 3 shows FFT analysis results of a pulse wave waveform and a pulse interval waveform;
Fig. 4 shows waveforms of bands HF, LF, VLF1, and VLF2 of the pulse wave waveform and pulse interval waveform;
Fig. 5 shows a signal correlation between the pulse wave waveform and the pulse interval waveform;
Fig. 6 shows an algorithm according to an embodiment of the present disclosure;
Fig. 7 shows a first application example of the algorithm according to the embodiment of the present disclosure;
Fig. 8 shows a second application example of the algorithm according to the embodiment of the present disclosure;
Fig. 9A shows a third application example of the algorithm according to the embodiment of the present disclosure;
Fig. 9B shows a third application example of the algorithm according to the embodiment of the present disclosure;
Fig. 10A shows a fourth application example of the algorithm according to the embodiment of the present disclosure;
Fig. 10B shows the fourth application example of the algorithm according to the embodiment of the present disclosure;
Fig. 11A shows a fifth application example of the algorithm according to the embodiment of the present disclosure;
Fig. 11B shows the fifth application example of the algorithm according to the embodiment of the present disclosure;
Fig. 12A shows a sixth application example of the algorithm according to the embodiment of the present disclosure;
Fig. 12B shows the sixth application example of the algorithm according to the embodiment of the present disclosure;
Fig. 13 shows an FFT analysis result of cerebral blood flow signals;
Fig. 14 shows a first proposal for a biological model of an autonomic nerve system;
Fig. 15 shows a second proposal for a biological model of the autonomic nerve system;
Fig. 16 shows a third proposal for a biological model of the autonomic nerve system;
Fig. 17 shows a fourth proposal for a biological model of the autonomic nerve system;
Fig. 18 shows an example of time series data (asynchronous/variable cycle) in a brain stem;
Fig. 19 is a conceptual diagram showing a 1/f filter;
Fig. 20 shows pulse waveforms and signal waveforms after being subjected to frequency filtering processing of each band;
Fig. 21 shows an FFT analysis result of a pulse wave waveform at 0.002 to 50 Hz;
Fig. 22 shows logarithmic amplitude distributions, normal distribution fitting lines, and distribution coefficients of HF (0.15 to 0.4 Hz), LF (0.04 to 15 Hz), VHF1 (1.5 to 4 Hz), VHF2 (0.4 to 1.5 Hz), UHF1 (15 to 40 Hz), and UHF2 (4 to 15 Hz) components of a pulse wave waveform;
Fig. 23 shows frequency distributions, normal distribution fitting lines, and distribution coefficients of HF (0.15 to 0.4 Hz), LF (0.04 to 0.15 Hz), VHF1 (1.5 to 4 Hz), VHF2 (0.4 to 1.5 Hz), UHF1 (15 to 40 Hz), and UHF2 (4 to 15 Hz) components of a pulse wave waveform;
Fig. 24 shows an example of an autonomic nerve index calculation system according to the embodiment of the present disclosure;
Fig. 25 shows an example of an autonomic nerve index calculation program according to the embodiment of the present disclosure;
Fig. 26 is a flowchart showing an example of processing performed in the autonomic nerve index calculation system according to the embodiment of the present disclosure; and
Fig. 27 shows LF/HF evaluation data according to a related method.

DESCRIPTION OF EMBODIMENTS

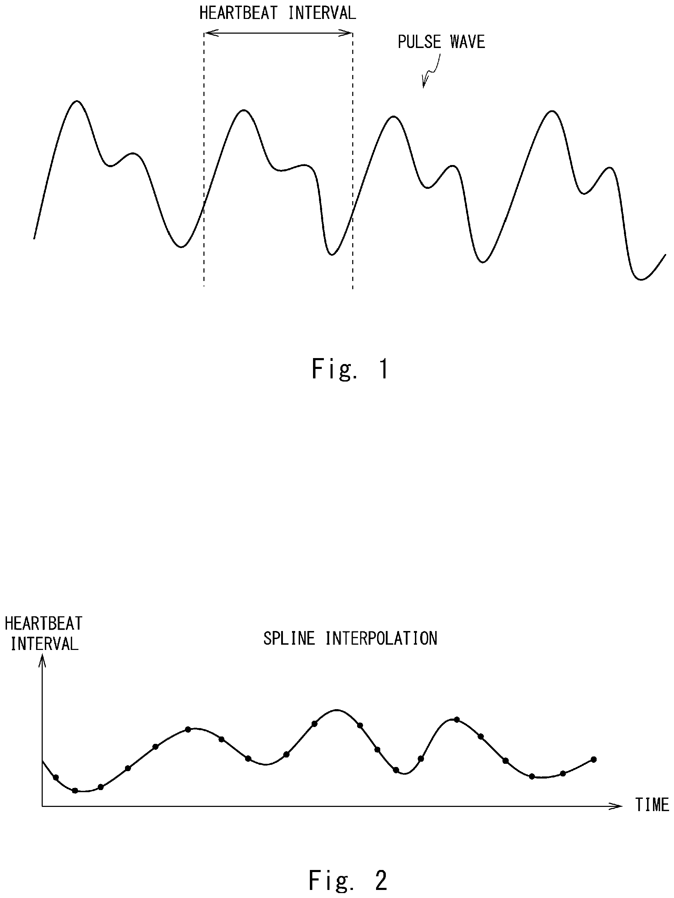

One embodiment of the present disclosure will now be described with reference to the drawings. A biometric sensor, such as a biosignals plux, is used to measure pulse waves of a subject in a steady state for several tens of minutes to several hours. The sampling frequency may be 500 Hz. The sampling frequency may be any frequency between 10 to 1000 Hz. The pulse wave waveform data B(t) thus obtained has a repetitive waveform as shown in Fig. 1. A pulse interval waveform R(t) as shown in Fig. 2 is obtained by calculating a pulse interval (RRI: R-R Interval) from the pulse wave waveform data B(t) using the pulse wave rising position detection algorithm presented in "An Open-source Algorithm to Detect Onset of Arterial Blood Pressure Pulses", W Zong, Harvard University-MIT Division of Health Sciences and Technology, and plotting rising times of the pulse wave and the pulse interval to perform spline interpolation.

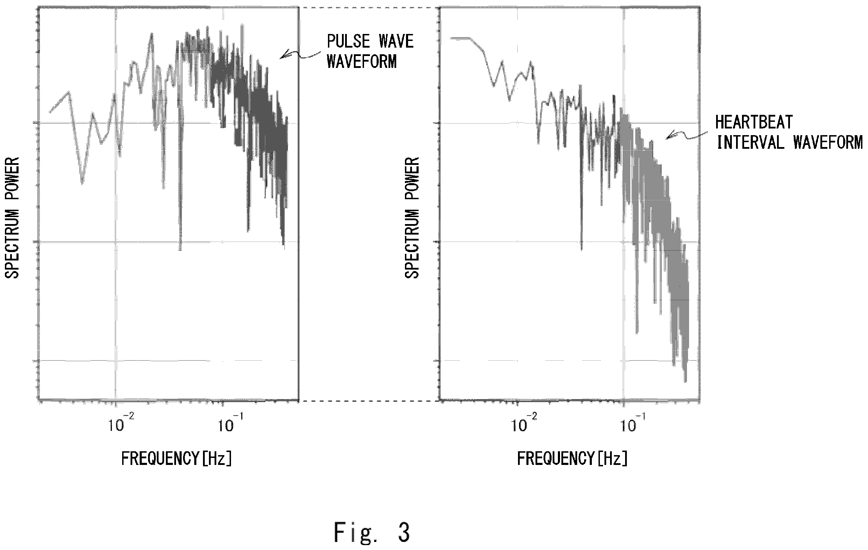

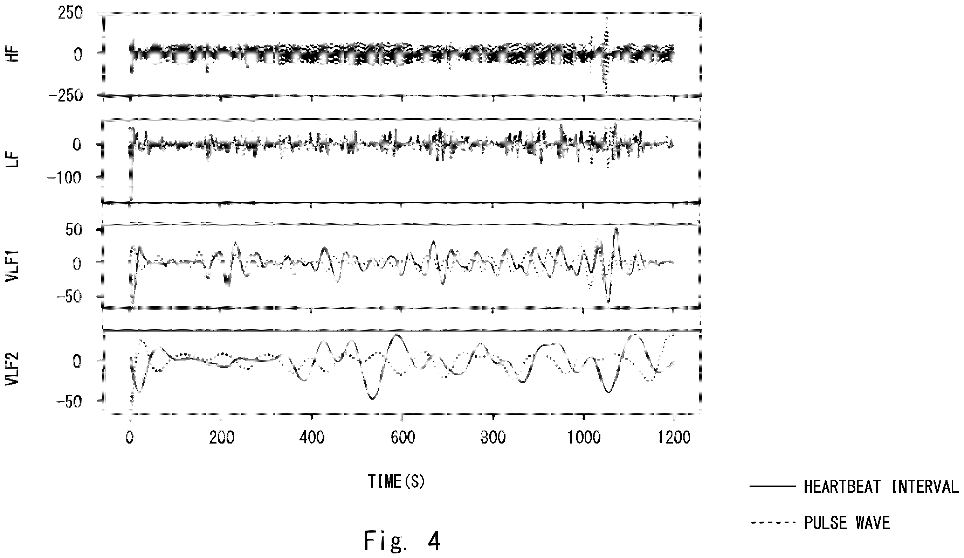

When the measured pulse wave waveform data B(t) and the pulse interval waveform data R(t) are subjected to FFT (Fast Fourier Transform) processing, a frequency spectrum as shown in Fig. 3 is obtained. In this embodiment, a frequency band is divided into HF (0.15 to 0.4 Hz), which is a respiratory fluctuation band of the heartbeat interval, LF (0.04 to 0.15 Hz), which is a blood pressure fluctuation, and VLF1 (0.015 to 0.04 Hz) and VLF2 (0.004 to 0.015 Hz), which are VLF (Very Low Frequency) bands. When the pulse wave waveform data B(t) and the pulse interval waveform R(t) are passed through a band-pass filter with four bands of HF, LF, VLF 1, and VLF2, waveforms of four bands as shown in Fig. 4 are obtained. The pulse wave waveform shown in Fig. 4 is data measured for 20 minutes in a sitting rest state.

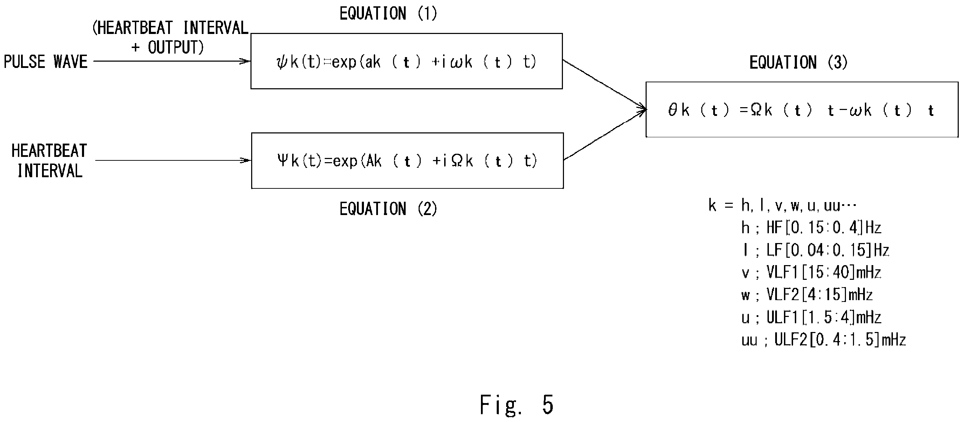

Next, the waveform of each band is subjected to Hilbert transformation to obtain a biological signal correlation diagram as shown in Fig. 5. The pulse interval is a biological signal constituted only by the pulse wave interval, while the pulse wave waveform is a biological signal waveform formed by the pulse interval, a blood output, and physical characteristics of blood vessels. As in Equations (1) and (2), for each band, the pulse interval waveform can be expressed using an amplitude term $A_k(t)$ and a phase term $S2_k(t)$, while the pulse wave waveform can be expressed using an amplitude term $a_k(t)$ and a phase term $\omega_k(t)$.

Referring to Fig. 3, in the VLF band of the pulse wave, the signal intensity rapidly decreases on a low frequency side due to a frequency characteristic of a pulse wave sensor, that is, an auto-range function. In order to compensate for this influence caused by the auto-range function, we focused on the phase term that is not easily affected by the decrease in the signal intensity, defined the phase difference shown in Equation (3), and focused on the amplitude term $A_k(t)$ and a phase difference $\theta_k$ of the pulse interval to proceed with the analysis of biological signals.

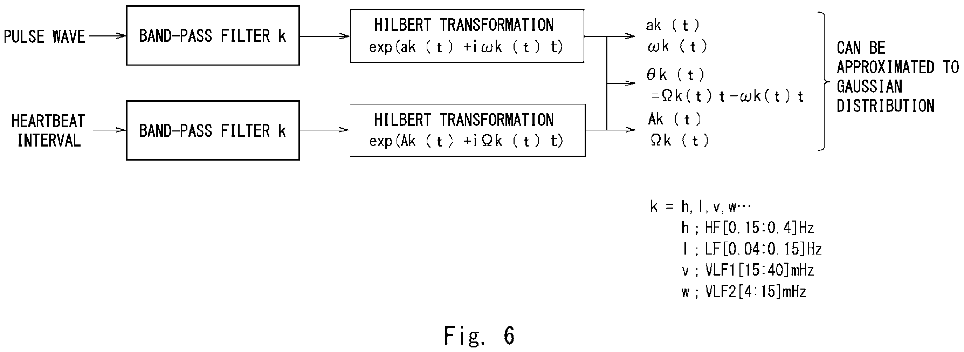

Fig. 6 is a conceptual diagram showing an example of the algorithm of a pulse wave waveform analysis program used in this experiment. After a pulse wave waveform and a pulse interval waveform are passed through a band-pass filter of four bands, each waveform signal is subjected to Hilbert transformation. Then, the amplitude terms $A_k(t)$ and $a_k(t)$ and phase terms $Q_k(t)$ and $\omega_k(t)$ of the complex waveform equation and a phase difference $\theta_k(t)$ between the pulse wave and the pulse interval are calculated, and these time series data are obtained. Here, 10 Hz can be employed as the sampling frequency. Also, any frequency between 5 to 200 Hz may be employed as the sampling frequency.

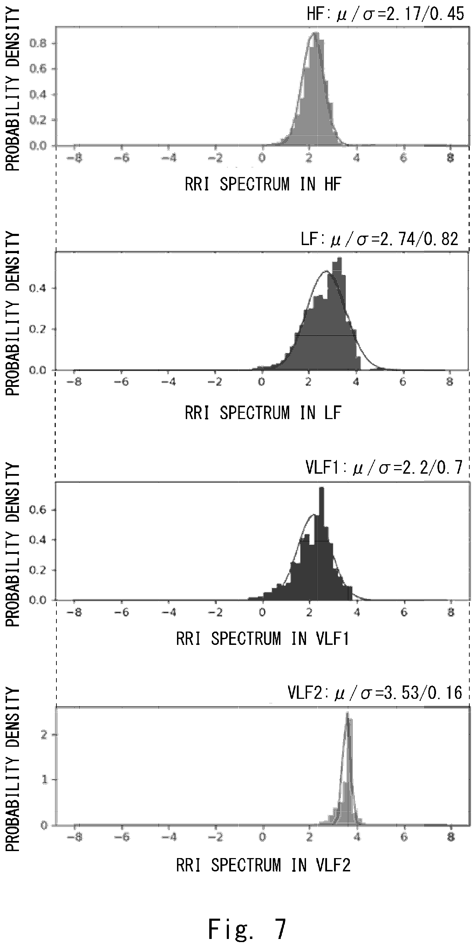

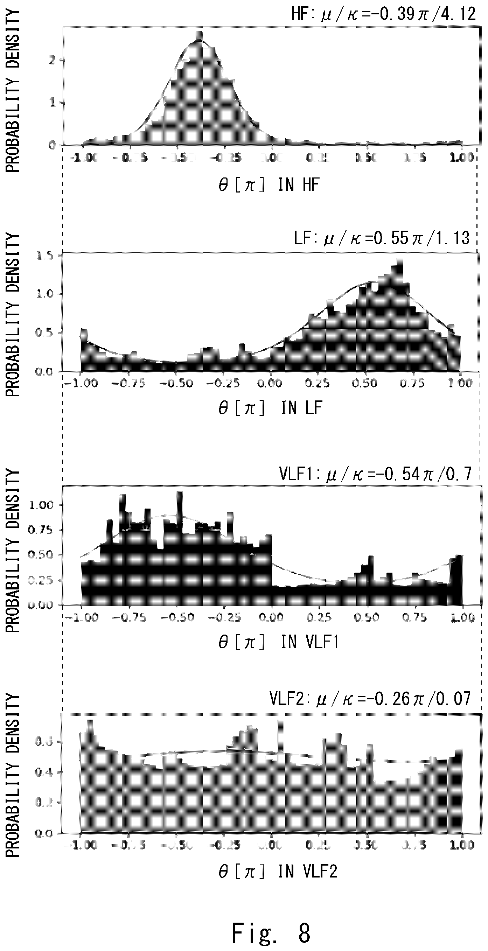

Figs. 7 and 8 show the distributions of the amplitude term $A_k(t)$ and the phase difference $\theta_k(t)$ of the pulse interval waveform obtained by processing the pulse wave data shown in Fig. 4 with the algorithm shown in Fig. 6. As shown in Fig. 7, it can be seen that the amplitude term $A_k$ is distributed like a Gaussian distribution (normal distribution) in all four bands. On the other hand, in Fig. 8, the phase difference $\theta_k$ between the pulse interval waveform and the pulse wave waveform is distributed in the Gaussian distribution (von Mises distribution) for $\theta_k$, k = h (HF), and l(LF), but for $\theta_k$, k = v(VLF 1), and w(VLF2), the phase difference $\theta_k$ does not appear to be distributed in the Gaussian distribution. The von Mises distribution can be expressed as Equation (4).

[Equation 4]

$$f(\theta k) = exp( \kappa cos(\theta k - \mu) ) / I_0(\kappa) \qquad (4)$$

Here, $I_0(\kappa)$ represents a Bessel function of order 0. $\mu$ represents a phase average and $\kappa$ represents a degree of concentration.

The curves in the graphs are the probability density distributions when the distributions are fitted to the Gaussian distribution, and the distribution coefficients of the respective distributions are also shown.

[Equation 5]

$$\theta = angle( \Sigma exp( i\theta k ) ) \qquad (5)$$

[Equation 6]

$$R = abs( \Sigma exp( i\theta k ) / N ) \qquad (6)$$

In this equation, i represents a complex number and N is the total number of samples. $\theta$ represents an average phase difference. R represents a degree of concentration.

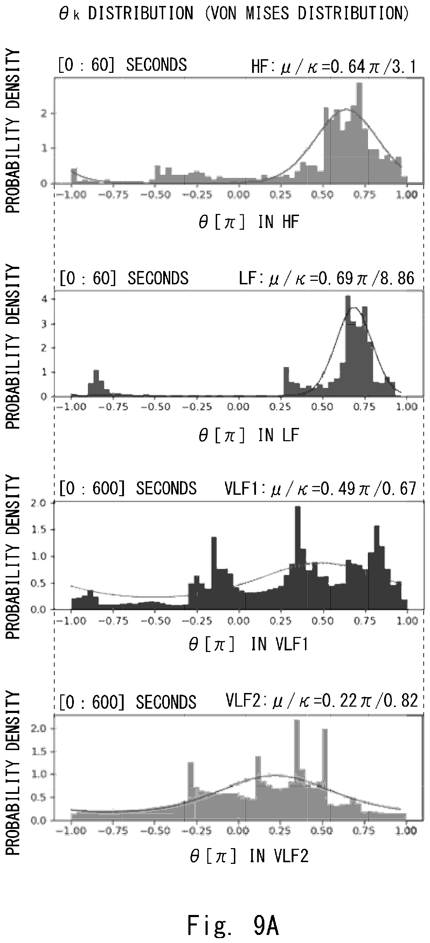

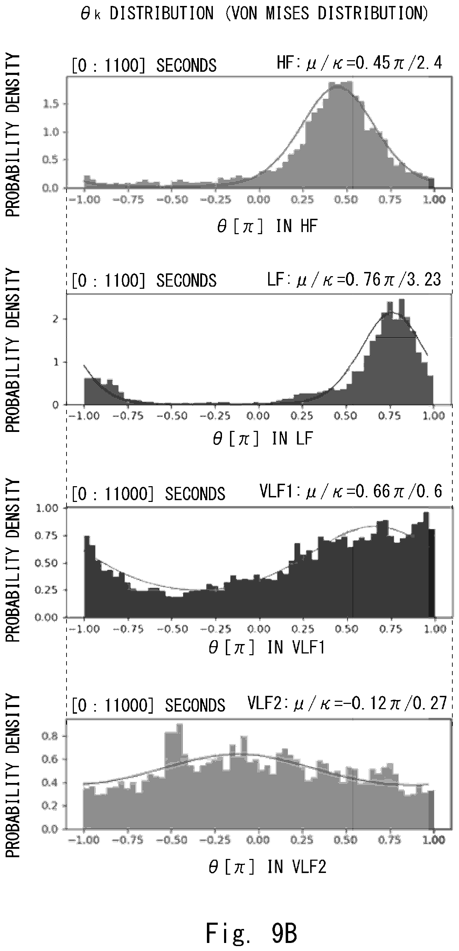

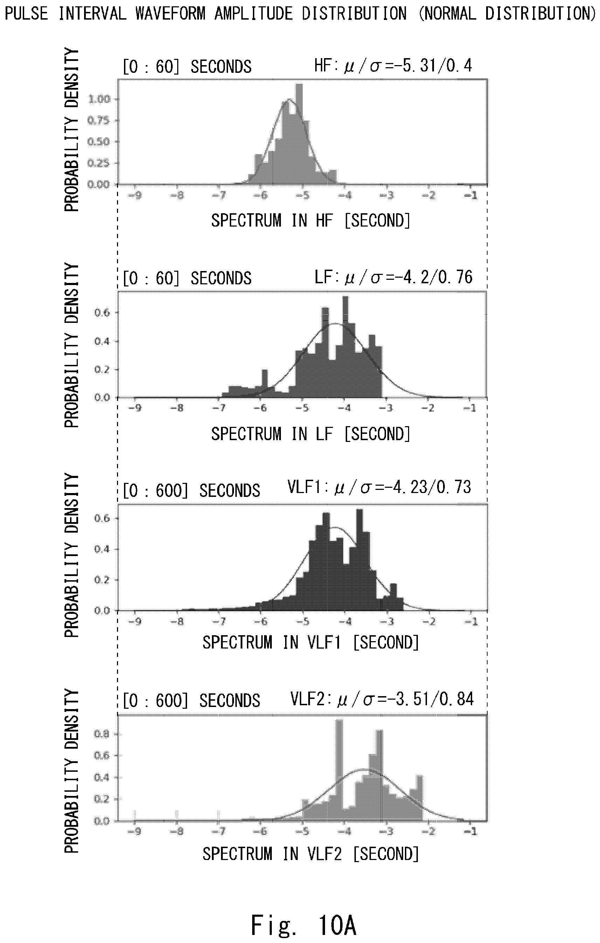

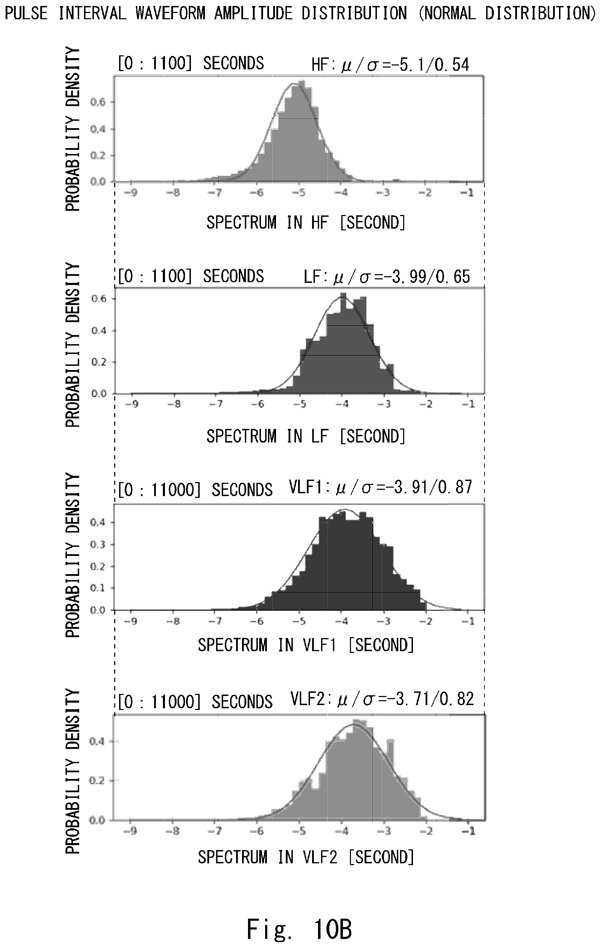

In order to confirm whether or not $\theta_k$ is in the Gaussian distribution in the VLF band, Figs. 9A, 9B, 10A, and 10B show results of viewing the distributions of the phase difference $\theta_k$ and the pulse interval amplitude term $A_k$ in the

data measured for three hours in a sitting rest state. Since a cycle of the VLF band is 10 times longer than that of the LF/HF band, the time interval must regarded as 10 times longer that of the LF/HF when the distribution of the VLF band is viewed. The distribution of the LF/HF is compared at time intervals of 60 seconds and 1,100 seconds with the distribution of the VLF 1/2 at time intervals of 600 seconds and 11,000 seconds. When the distribution of the VLF 1/2 in 11,000 seconds is compared with the distribution of the LF/HF in 1,100 seconds which corresponds to 11,000 seconds of the VLF 1/2, it is found that all four bands can be approximated by the Gaussian distribution (von Mises distribution).

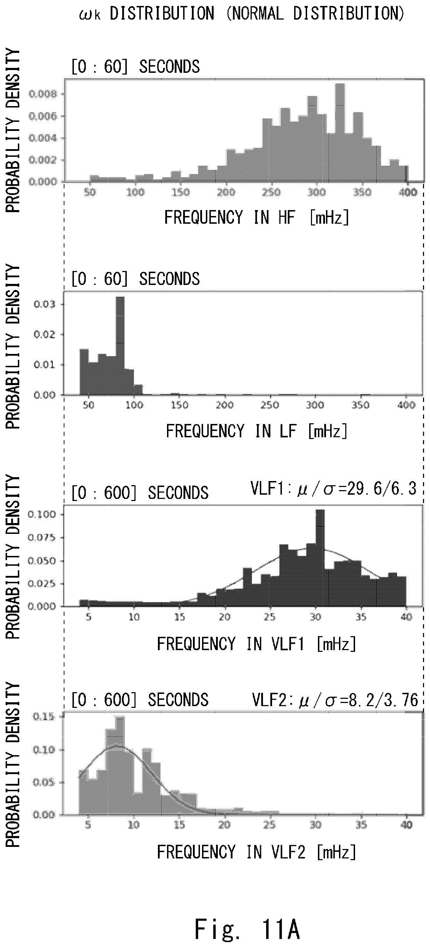

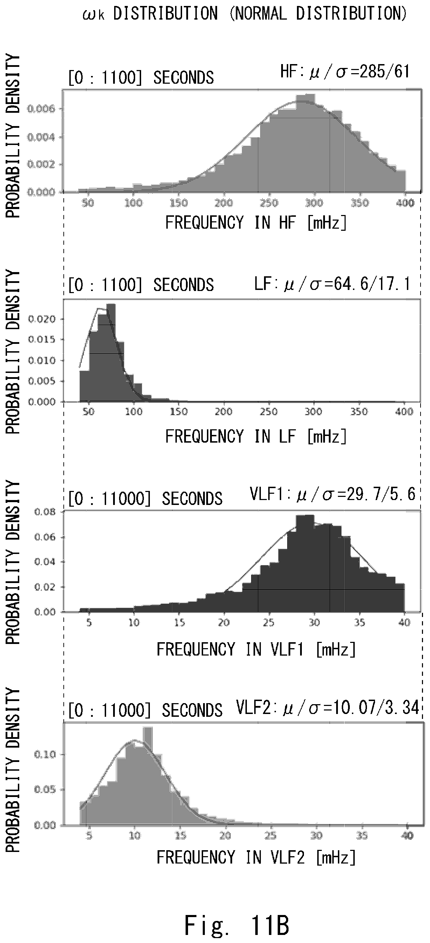

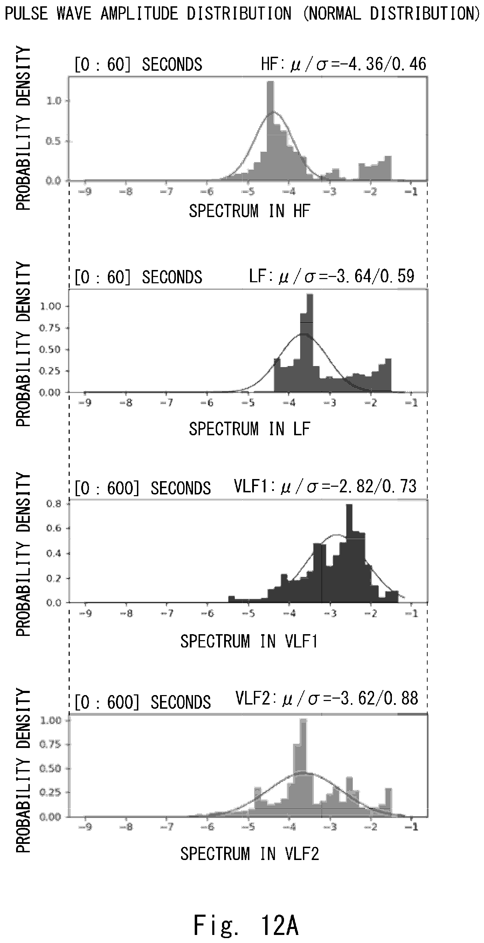

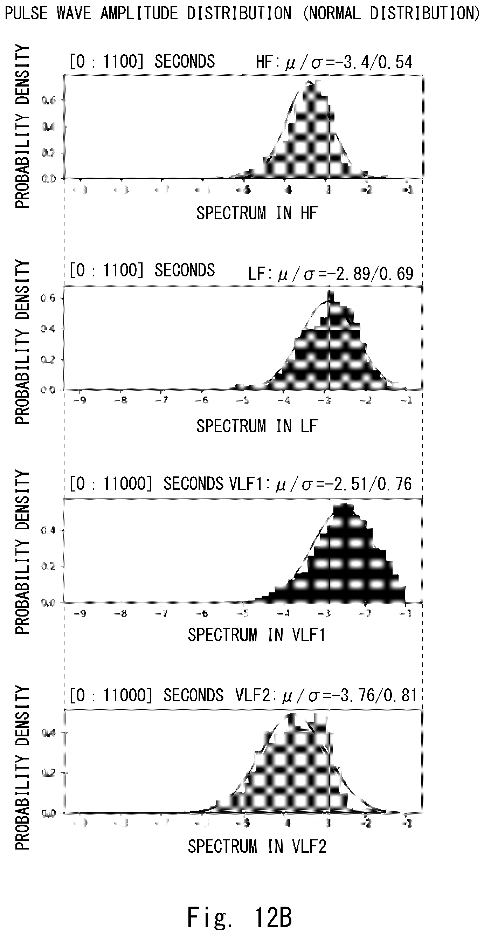

According to the biological signal correlation diagram of Fig. 5, since the amplitude term Ak and the phase difference $\theta$k of the pulse interval waveform are distributed like the Gaussian distribution, it is possible that the amplitude term ak and the phase term $\omega$k of the pulse wave waveform are also distributed like the Gaussian distribution. As a result of confirming the distribution of the amplitude term ak and the phase term $\omega$k of the pulse wave waveform by the pulse wave data of three hours in a sitting rest state, it is found that ak and $\omega$k can be approximated by the Gaussian distribution as shown in Figs. 11A, 11B, 12A, and 12B.

By processing the pulse wave data when a living body is in a steady state using the algorithm shown in Fig. 6, and extracting at least one each of feature amounts of the amplitude terms (Ak, ak) and the phase terms ($\theta$k, $\omega$k, S2k), namely, at least two feature amounts, which are independent of each other, for four bands, it is possible to accurately describe the features of biological responses of, for example, respiration, blood pressure, and autonomic nerves to various environmental stimuli. In a manner similar to this, by extracting the feature amounts for the ULF band having a frequency lower than that of the VLF band and a cycle longer than that of the ULF band, the feature amounts of the biological response to more environmental stimuli can be described.

When the living body is in the steady state, it is found that the feature amount extracted by the algorithm of Fig. 6 can be approximated by the Gaussian distribution, so even if a measurement time is short and a data length necessary for the band cannot be obtained, each feature amount can be estimated by performing statistical processing. Although the description has been made on the case where each feature amount has the Gaussian distribution as an example, a distribution other than the Gaussian distribution (e.g., Poisson distribution or the like) can be applied.

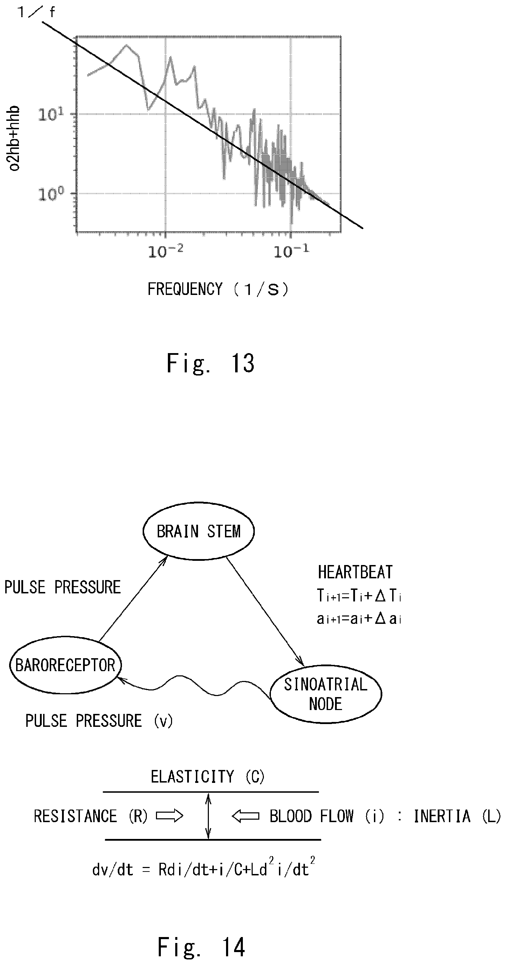

The pulse wave frequency spectrum shown in Fig. 3 has a problem that a sufficient signal intensity cannot be obtained in a frequency band below the VLF range due to the influence of the frequency characteristic (auto-range function, cutoff frequency 0.2 Hz) of a pulse wave sensor. In the future, we plan to obtain a pulse wave sensor without the auto-range function to measure pulse waves, and apply this algorithm. Fig. 13 shows results of an FFT analysis of data obtained by measuring blood flow concentration in the frontal lobe using a tNIRS-1 non-invasive cerebral oxygen monitor manufactured by Hamamatsu Photonics K.K. In Fig. 13, o2hb is an oxygenated hemoglobin concentration and hhb is a deoxygenated hemoglobin concentration. Further, o2hb+hhb is proportional to an amount of frontal lobe blood flow.

Referring to Fig. 13, a frequency spectrum of a total hemoglobin concentration (o2hb + hhb) is on a 1/f line. Therefore, it can be considered that a fluctuation spectrum of the amount of the blood flow is on 1/f.

Fig. 14 is a schematic diagram of a model of heart rate variability. The brain stem includes a nerve center for controlling a heart rate, and this nerve center sends signals for controlling heartbeat intervals and the blood output to, for example, the sinoatrial node in the right atrium of the heart. The pulse wave of blood is sent to the whole body, including the carotid artery, at the corresponding heartbeat interval and output. The pulse wave follows physical property value of blood vessels, and using a blood flow i, inertia L of the blood, elasticity C of the blood vessels, and a resistance R of the blood vessels, a pulse pressure v can be expressed as in the following second-order differential equation.
[Equation 7]

$$dv/dt = Rdi/dt + i/C + Ld^2i/dt^2 \qquad (7)$$

Once the heartbeat interval and blood output are determined, the pulse pressure v can be obtained from baroreceptors in the carotid artery by solving this differential equation. The pulse pressure detected by the baroreceptors is transmitted to the brain stem, and the next heartbeat interval and blood output are calculated by the brain stem, and a result of the calculation is sent to the sinoatrial node.

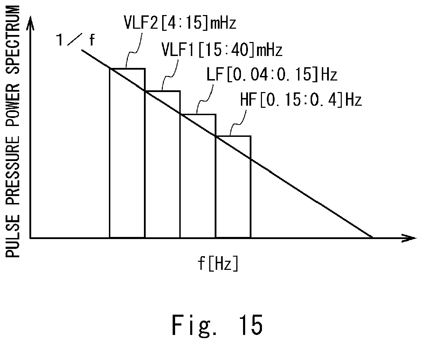

The brain stem must determine the heartbeat interval and blood output to generate the wave function (1) for all bands shown in Fig. 15 (including signals with cycles longer than that of the VLF band).

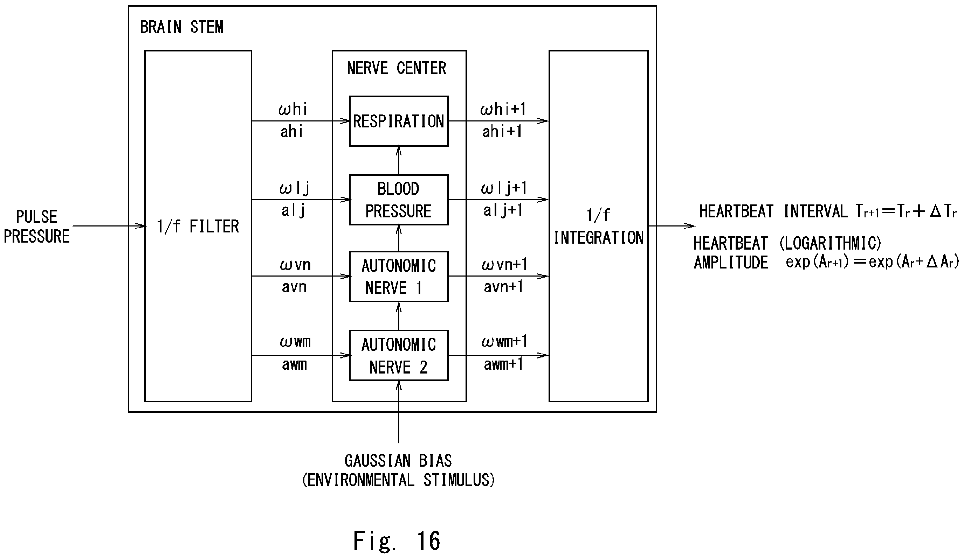

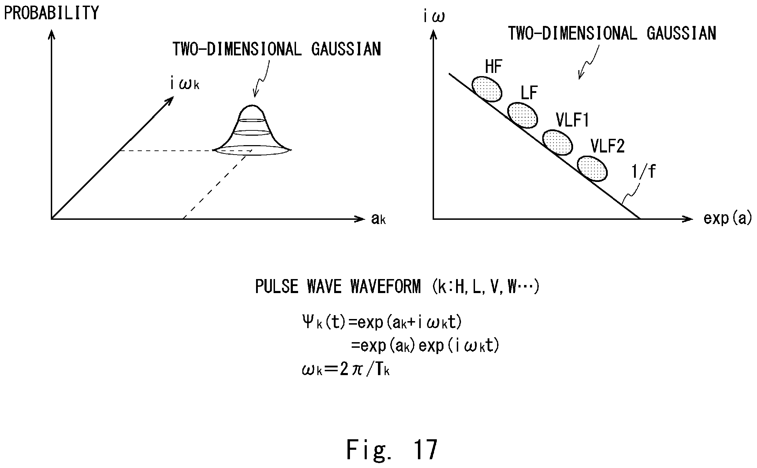

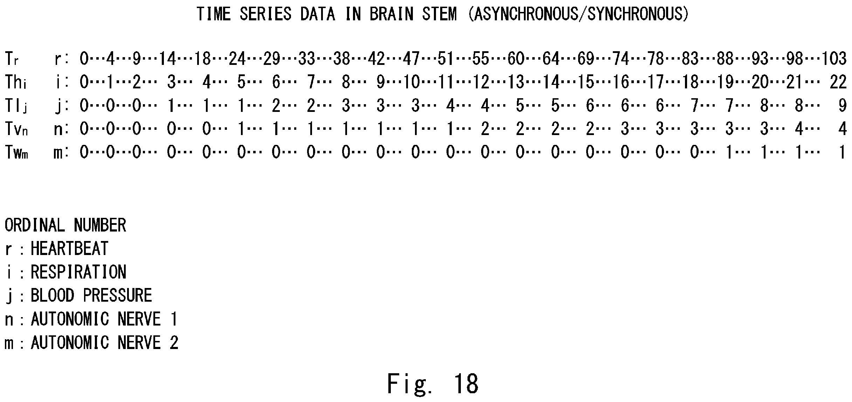

Based on the experimental data so far, the content of the calculation in the brain stem is estimated and shown in Fig. 16. The pulse pressure data sent from the baroreceptors is separated into a pulse pressure cycle and a pulse pressure amplitude term for each band by a 1/f filter (which will be described later), and allocated to nerve centers of, for example, respiration, blood pressure, and autonomic nerves. The pulse pressure frequencies ($\omega$h$_i$, $\omega$l$_j$, $\omega$v$_n$, $\omega$w$_m$, ... where $\omega$k=2$\pi$/Tk k:h, l, v, w,...) and pulse pressure amplitudes (ahi, al$_j$, av$_n$, aw$_m$, ...) allocated to the respective bands are input to the nerve centers of the respiratory, blood pressure, and autonomic nerve system in response to an environmental stimulus (tension, relaxation, fear, fatigue, alertness, warmth, coldness etc.), a mean value and a variance value of a two-dimensional Gaussian distribution (Fig. 17) of the pulse pressure frequency and pulse pressure amplitude term in response to the stimulus are set (Gaussian bias), and the following pulse pressure frequencies ($\omega h_{i+1}$, $\omega l_{j+1}$, $\omega v_{n+1}$, $\omega w_{m+1}$, ...) and pulse pressure amplitude terms ($ah_{i+1}$, $al_{j+1}$, $av_{n+1}$, $aw_{m+1}$, ...) are set to achieve the values. These signals are input to the 1/f integration, where an inverse process of the 1/f filter is processed to generate the next heartbeat interval $T_{r+1}=T_r+\Delta T_r$ and pulse wave amplitude $\exp(A_{r+1})=\exp(A_r+\Delta A_r)$ which are sent to the sinoatrial node to control the heartbeat interval and the blood output. Here, the cycle is different for each band (Tk k:h,l,v,w,... Tk=$2\pi/\omega$k), and the time series data of the heartbeat interval $T_r$ and the cycle Tk of each band is a data series of an asynchronous variable cycle as shown in Fig. 18.

The Gaussian bias of Fig. 16 will be described using switching of the respiratory fluctuation and the blood pressure fluctuation as an example. Relaxation causes respiratory heart rate variability. In the Gaussian bias input to the respiratory center at that time, the variance of the two-dimensional Gaussian of HF is small and the HF pulse pressure amplitude is large. The Gaussian bias input to the blood pressure center which contributes to the blood pressure fluctuation generates time series data of each pulse pressure amplitude term and pulse pressure frequency term so that the variance of the two-dimensional Gaussian of LF becomes large and the LF pulse pressure amplitude becomes small. As a result, the respiratory heart rate variability (RSA) appears as a physiological phenomenon.

Tension causes hypertensive heart rate variability. In the Gaussian bias input to the respiratory center at that time, the variance of the two-dimensional Gaussian of HF is large and the HF pulse pressure amplitude is small. The Gaussian bias input to the blood pressure center which contributes to the blood pressure fluctuation generates time series data of each pulse pressure amplitude term and pulse pressure frequency term so that the variance of the two-dimensional Gaussian of the LF becomes small and the LF pulse pressure amplitude becomes large. As a result, the blood pressure related heart rate variability appears as a physiological phenomenon.

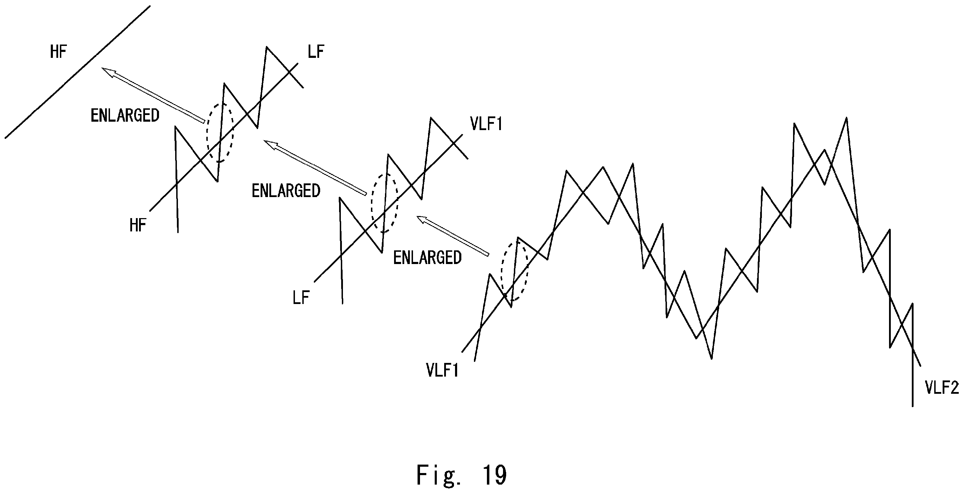

The 1/f filter of Fig. 16 will be described. Fig. 19 schematically shows waveforms of pulse pressures of HF (0.15 to 0.4 Hz), LF (0.04 to 0.15 Hz), VLF1 (0.015 to 0.04 Hz), and VLF2 (0.004 to 0.015 Hz). Each band waveform has a fractal structure. When a time axis and an amplitude axis corresponding to each band are set, they all have the same structure, and a signal of the band can be received only by setting a time constant of a receiver of the signal according to each band. In addition, the line of 1/f in Fig. 15 represents wave energy = constant (hv = constant, h: Planck constant, v: frequency) in quantum mechanics, which may be interpreted as indicating the lowest pulse pressure wave energy detectable by the living body. Therefore, it can be said that the energy efficiency is the best for the transmission of pulse pressure wave energy.

The 1/f integration in Fig. 16 is an inverse process of the system from the sinoatrial node in Fig. 14 through the baroreceptors to the 1/f filter in the brain stem, and can be considered as the transfer function that determines the heartbeat interval $T_{r+1}$ and the blood output $\exp(A_{r+1})$ to achieve the Gaussian biased $ak_{p+1}$, $\omega k_{p+1}$ (k:h, l, v, w,... p:i, j, n, m) calculated by the nerve centers of, for example, respiration, blood pressure, and the autonomic nerve system in the brain stem. A functional form of the transfer function is unknown, but it is presumed to be implemented by neural networks that are formed in the brain stem (environmental adaptation) from the time a new born is exposed to various environmental stimuli and becomes an adult.

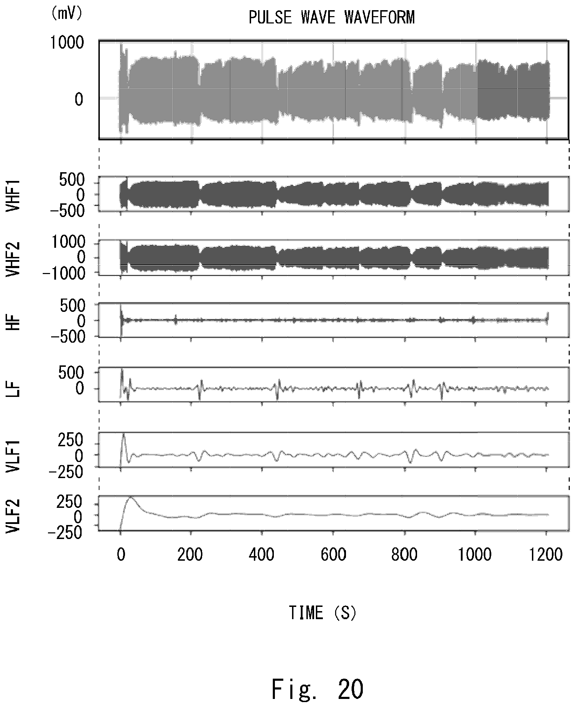

In the above embodiment, the pulse wave waveform or heartbeat interval waveform is divided into four bands of HF, LF, VLF1, and VLF2 to perform the data processing. Similar data processing is performed on frequencies higher than 0.4 Hz. Fig. 20 shows waveforms after band-pass filtering processing of two bands of VHF 1 (1.5 to 4 Hz) and VHF 2 (0.4 to 1.5 Hz) in addition to pulse wave waveforms and four bands of the embodiment.

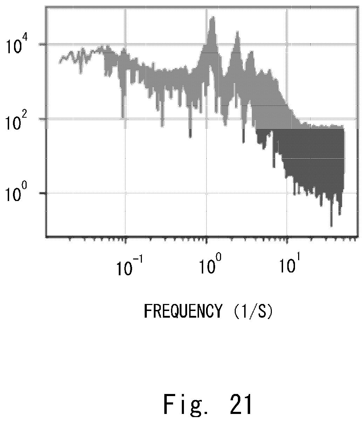

When this pulse wave waveform is subjected to FFT processing in the range of 0.002 to 50 Hz, the pulse wave waveform becomes the one shown in Fig. 21 and it can be seen that there is a data structure in the frequency range of 0.4 to 40 Hz. This frequency range corresponds to the frequency range of the electroencephalogram ($\delta$, $\theta$, $\alpha$, and $\beta$ waves).

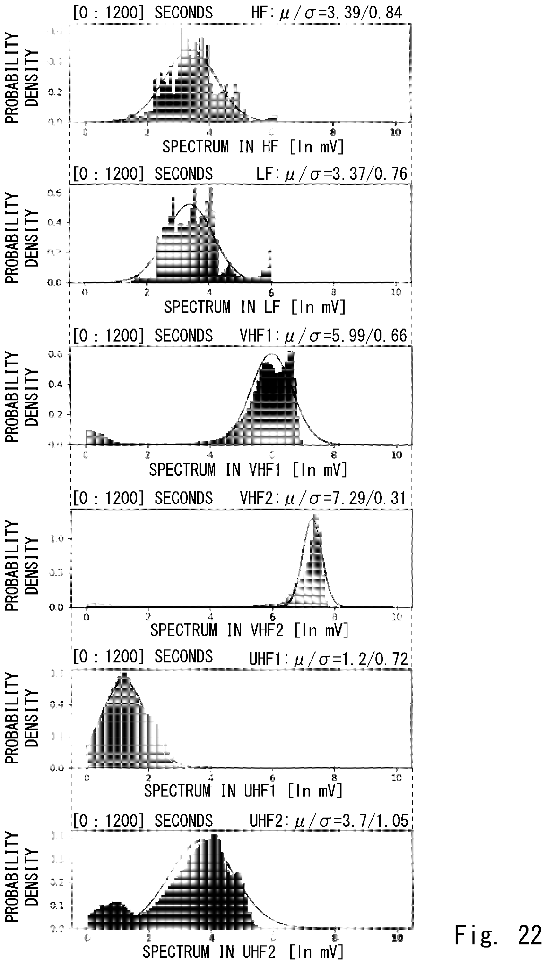

The results of similar information processing in these frequency bands are shown below. Logarithmic amplitude terms are extracted for four bands of VHF 1 (1.5 to 4 Hz), VHF 2 (0.4 to 1.5 Hz), UHF 1 (15 to 40 Hz), and UHF 2 (4 to 15 Hz), and histograms, normal distribution fitting lines, and distribution coefficients of the four bands are shown in Fig. 22.

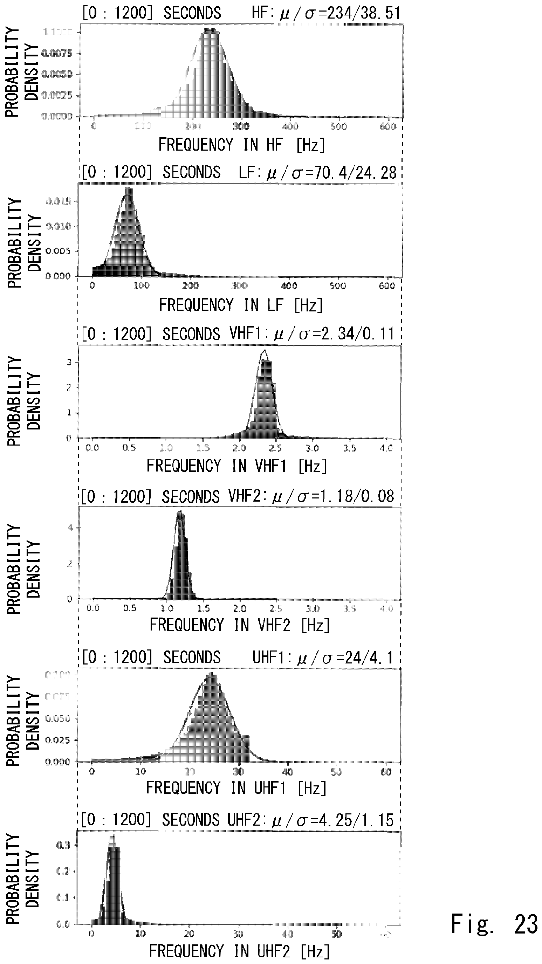

Furthermore, frequency terms are extracted for four bands of VHF 1 (1.5 to 4 Hz), VHF 2 (0.4 to 1.5 Hz), UHF 1 (15 to 40 Hz), and UHF 2 (4 to 15 Hz), and histograms, normal distribution fitting lines, and distribution coefficients of the four bands are shown in Fig. 23. It can be seen that a physiological state in these frequency bands can also be described by the distribution coefficient of each band.

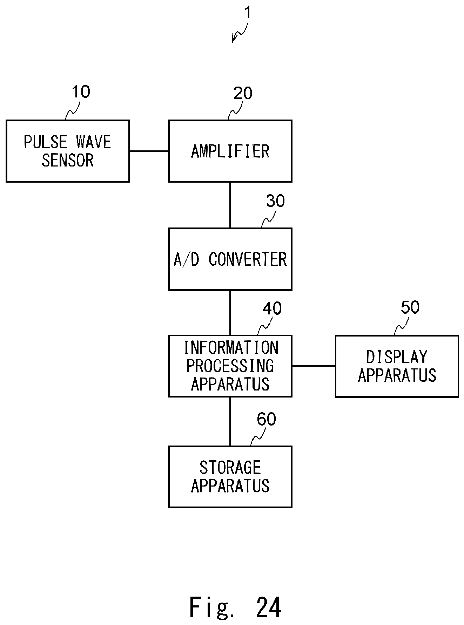

Fig. 24 shows an example of an autonomic nerve index calculation system 1 according to the embodiment of the present disclosure. The autonomic nerve index calculation system 1 includes a pulse wave sensor 10, an amplifier 20, an A/D converter 30, an information processing apparatus 40, a display 50, and a storage apparatus 60. The autonomic nerve index calculation system 1 can be configured as one apparatus including these apparatuses. In this case, specific examples of the autonomic nerve index calculation system 1 include a PC (Personal Computer), a raspberry pi, a smartphone, a wearable terminal, a tablet terminal, or the like.

In addition, the autonomic nerve index calculation system 1 may implement these apparatuses as individual apparatuses. For example, the autonomic nerve index calculation system 1 can be configured as a system including an apparatus provided with the pulse wave sensor 10, the amplifier 20, and the A/D converter 30, and an apparatus provided with the information processing apparatus 40, the display 50, and the storage apparatus 60. Alternatively, the autonomic nerve index calculation system 1 can be configured as a system including an apparatus provided with the pulse wave sensor 10, the amplifier 20, and the A/D converter 30, and an apparatus provided with the information processing apparatus 40, the display 50, and the storage apparatus 60. These apparatuses can communicate with each other via a network that can be formed by a LAN (Local Area Network) and/or a WAN (Wide Area Network). Further, communication between these apparatuses can be performed via a wire or wirelessly.

The pulse wave sensor 10 is an apparatus for detecting a pulse wave from the living body. The pulse wave sensor 10 provides a pulse wave signal that is time series data indicating the detected pulse wave to the amplifier 20. Although Fig. 24 shows a single pulse wave sensor 10, the autonomic nerve index calculation system 1 may include a plurality of the pulse wave sensors 10.

The amplifier 20 amplifies the pulse wave signal provided from the pulse wave sensor 10. The A/D converter 30 converts the pulse wave signal as an analog signal provided from the amplifier 20 into a digital signal, and provides the digitized pulse wave signal to the information processing apparatus 40. The A/D converter 30 provides the pulse wave signal, which is time-series data, to the information processing apparatus 40.

The information processing apparatus 40 calculates an autonomic nerve index of the living body using the pulse wave signal provided from the A/D converter 30. Specific examples of the information processing apparatus 40 include a PC, a CPU (Central Processing Unit), an MPU (Micro Processing Unit), an ECU (Electronic Control Unit), an FPGA (Field-Programmable Gate Array), an ASIC (Application Specific Integrated Circuit), and the like. The information processing apparatus 40 implements an autonomic nerve index calculation method by executing an autonomic nerve index calculation program described later. The information processing apparatus 40 displays the calculated autonomic nerve index on the display 50.

The display 50 is an apparatus for displaying various information such as the autonomic nerve index calculated by the information processing apparatus 40. The storage apparatus 60 stores various kinds of information such as an autonomic nerve index calculation program.

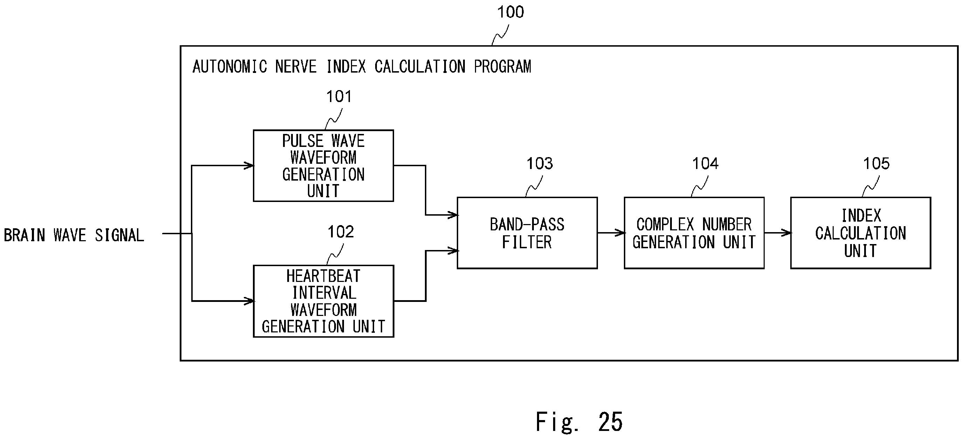

Fig. 25 is a diagram showing an example of an autonomic nerve index calculation program 100. The autonomic nerve index calculation program 100 includes a pulse wave waveform generation unit 101, a heartbeat interval waveform generation unit 102, a band-pass filter 103, a complex number generation unit 104, and an index calculation unit 105.

The pulse wave waveform generation unit 101 is a program module for generating pulse wave waveform data using at least one pulse wave signal provided from the A/D converter 30. The heartbeat interval waveform generation unit 102 is a program module for generating the heartbeat interval waveform data using at least one pulse wave signal provided from the A/D converter 30.

The band-pass filter 103 is a program module for filtering the pulse wave waveform data generated by the pulse waveform generation unit 101 and the heartbeat interval waveform data generated by the heartbeat interval waveform generation unit 102. More specifically, the band-pass filter 103 applies FFT conversion to at least one of the pulse wave waveform data and the heartbeat interval waveform data, and divides the band of the converted pulse wave waveform data and the converted heartbeat interval waveform data by at least one predetermined frequency band. The predetermined frequency bands include, for example, bands such as 0.004 to 0.015 Hz (VLF2), 0.015 to 0.04 Hz (VLF1), 0.04 to 0.15 Hz (LF), 0.15 to 0.4 Hz (HF), 0.4 to 1.5 Hz (VHF2), 1.5 to 4 Hz (VHF1), 4 to 15 Hz (UHF2), and 15 to 40 Hz (UHF1).

The complex number generation unit 104 is a program module for converting at least one of the pulse wave waveform data and the heartbeat interval waveform data filtered by the band-pass filter 103 into complex numbers. Specifically, the complex number generation unit 104 converts the filtered pulse wave waveform data into a complex number and calculates the complex pulse wave waveform data (hereinafter referred to as "pulse wave complex waveform data"). The complex number conversion unit 104 converts the filtered heartbeat interval waveform data into a complex number and calculates the complex heartbeat interval waveform data (hereinafter referred to as "heartbeat interval complex waveform data"). The complex number conversion unit 104 employs Hilbert transformation as a complex number conversion method. In another example not explicitly set out in the claims, the complex number conversion method is not limited to the Hilbert transformation, and any complex number conversion method may be employed.

The index calculation unit 105 is a program module for calculating the autonomic nerve index of the living body based on at least one of the pulse wave complex waveform data and the heartbeat interval complex waveform data. Specifically, the index calculation unit 105 can calculate an instantaneous amplitude corresponding to an absolute value of the amplitude term $A_k(t)$ of the pulse wave complex waveform data as the autonomic nerve index. Moreover, the index calculation unit 105 can calculate an instantaneous frequency corresponding to a time differential value of the phase term $\omega_k(t)$ of the pulse wave complex waveform data as the autonomic nerve index.

Further, the index calculation unit 105 can calculate the distribution of the instantaneous amplitude based on the pulse wave complex waveform data at predetermined time intervals, and calculate the distribution coefficient based on the distribution of the instantaneous amplitude of the pulse wave complex waveform data as the autonomic nerve index. Furthermore, the index calculation unit 105 can calculate the distribution of the instantaneous frequency based on the pulse wave complex waveform data at predetermined time intervals, and calculate the distribution coefficient based on the distribution of the instantaneous frequency of the pulse wave complex waveform data as the autonomic nerve index.

In addition, the index calculation unit 105 can calculate an instantaneous amplitude corresponding to an absolute value of the amplitude term Ak(t) of the heartbeat interval complex waveform data as the autonomic nerve index. Further, the index calculation unit 105 can calculate an instantaneous frequency corresponding to a time differential value of the phase term S2k(t) of the heartbeat interval complex waveform data as the autonomic nerve index.

Furthermore, the index calculation unit 105 can calculate the distribution of the instantaneous amplitude based on the heartbeat interval complex waveform data at predetermined time intervals, and calculate the distribution coefficient based on the distribution of the instantaneous amplitude of the heartbeat interval complex waveform data as the autonomic nerve index. In addition, the index calculation unit 105 can calculate the distribution of the instantaneous frequency based on the heartbeat interval complex waveform data at predetermined time intervals, and calculate the distribution coefficient based on the distribution of the instantaneous frequency of the heartbeat interval complex waveform data as the autonomic nerve index.

Further, the index calculation unit 105 can calculate the instantaneous phase difference θk(t), which is the difference between the phase terms of the pulse wave complex waveform data and the heartbeat interval complex waveform data, as the autonomic nerve index. The instantaneous phase difference θk(t) can be derived by subtracting the phase term ωk(t)t of the pulse wave complex waveform data from the phase term S2k(t)t of the heartbeat interval complex waveform data, as shown in Equation 3.

Furthermore, the index calculation unit 105 can calculate the distribution of the instantaneous phase difference θk(t) at predetermined time intervals, and calculate the distribution coefficient based on the distribution of the instantaneous phase difference θk(t) as the autonomic nerve index.

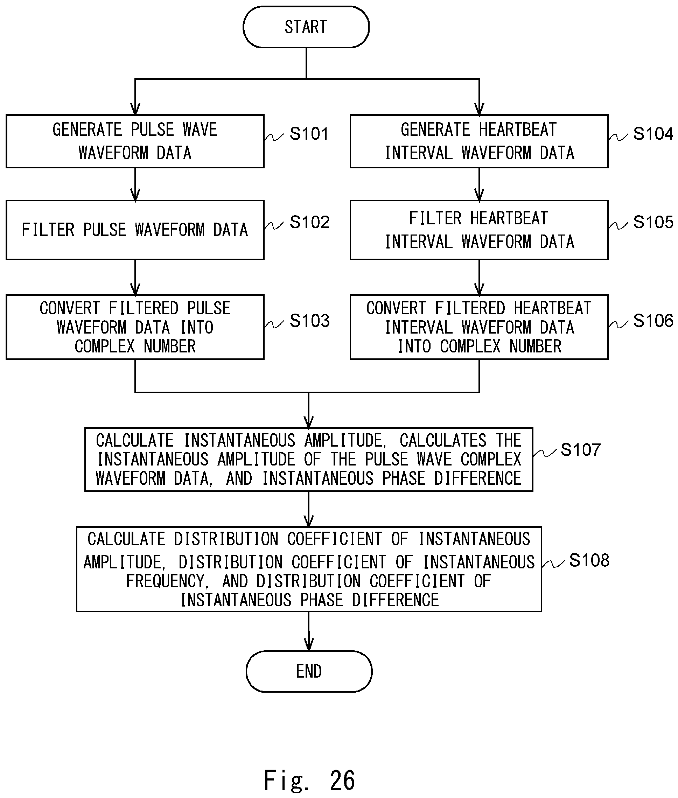

Fig. 26 is a flowchart showing an example of processing executed in the autonomic nerve index calculation system 1 according to an embodiment of the present disclosure.

In Step S101, the pulse wave waveform generation unit 101 generates the pulse wave waveform data using at least one pulse wave signal. In Step S102, the band-pass filter 103 filters the pulse wave waveform data in at least one frequency band. In Step S103, the complex number generation unit 104 converts the filtered pulse wave waveform data into a complex number to generate the pulse complex waveform data in at least one frequency band.

In Step S104, the heartbeat interval waveform generation unit 102 generates the heartbeat interval waveform data using at least one pulse wave signal. In Step S105, the band-pass filter 103 filters the heartbeat interval waveform data in at least one frequency band. In Step S106, the complex number generation unit 104 converts the filtered heartbeat interval waveform data into a complex number to generate heartbeat interval complex waveform data in at least one frequency band.

In Step S107, the index calculation unit 105 calculates the instantaneous amplitude of the pulse wave complex waveform data, the instantaneous frequency of the pulse wave complex waveform data, the instantaneous amplitude of the heartbeat interval complex waveform data, the instantaneous frequency of the heartbeat interval complex waveform data, and the instantaneous phase difference θk(t) between the pulse wave complex waveform data and the heartbeat interval complex waveform data using the pulse wave complex waveform data and the heartbeat interval complex waveform data in at least one frequency band. In Step S108, the index calculation unit 105 calculates, at predetermined time intervals, the distribution coefficient of the instantaneous amplitude of the pulse wave complex waveform data, the distribution coefficient of the instantaneous frequency of the pulse wave complex waveform data, the distribution coefficient of the instantaneous amplitude of the heartbeat interval complex waveform data, the distribution coefficient of the instantaneous frequency of the heartbeat interval complex waveform data, and the distribution coefficient of the instantaneous phase difference θk(t) for at least one frequency band using the information calculated in Step S107. Then, the processing of Fig. 26 is ended.

In the processing shown in Fig. 26, the distribution coefficient of the instantaneous amplitude and the distribution coefficient of the instantaneous frequency of the pulse wave complex waveform data, the distribution coefficient of the instantaneous amplitude and the distribution coefficient of the instantaneous frequency of the heartbeat interval complex waveform data, and the distribution coefficient of the instantaneous phase difference θk(t) are calculated as the autonomic nerve index. However, the autonomic nerve index calculation system 1 can calculate at least one of these distribution coefficients as the autonomic nerve index.

As an information processing method in the frequency bands (VHF1, VHF2, UHF1, UHF2, etc.) higher than 0.4 Hz, the processing shown in Fig. 26 may be executed using a brain wave waveform instead of the heartbeat interval waveform. Since a brain wave amplitude is proportional to a firing frequency of neurons and is considered to be proportional to a cerebral blood flow, the physiological state may be further described in detail by mathematically describing the interaction between the pulse wave waveform of a brain wave band and a brain wave. The index describing the physiological state can be calculated by obtaining the distribution coefficient of the amplitude term and the phase difference term of the pulse wave waveform and the brain wave waveform.

The function of the right brain is different from the function of the left brain, and of course an amount of the blood flow supplied to the right brain is different from the blood flow supplied to the left brain. When the cerebral blood flow is actually measured using an apparatus such as NIRS, the cerebral blood flow in the right brain differs from that in the left brain. Thus, regarding the carotid arteries supply blood to the brain, the blood flow in the right carotid arteries differs from that in the left carotid arteris. A plurality of pulse wave sensors are attached to the right and left carotid arteries, ears and temples to measure pulse waves, and an index describing the physiological state related to the left and right pulse waves and brain waves can be calculated.

In addition, a similar analysis is possible for frequencies below 0.004 Hz (e.g., 0.0004 Hz to 0.004 Hz). For frequencies in this range, it is possible to perform measurement for a very long time (several tens of hours to several months) and calculate the autonomic nerve index using the measured data.

In addition to the function of the autonomic nerve system, the physiological state of the living body includes fluctuations with very long cycles including diurnal fluctuation, weekly fluctuation, and seasonal fluctuation such as those in the immune system. As for cycles shorter than the autonomic nerve system, there are brain waves such as $\delta$, $\theta$, $\alpha$, $\beta$, and $\gamma$ waves. The index of physiological state proposed in this paper presents the index of these various physiological states in a comprehensive and unified way.

The pulse wave (pulse pressure) $\psi(t)$ representing the physiological state can be expressed by a linear combination of frequency bands k as follows.

[Equation 8]

$$\psi\ (\ t\ )\ =\ \Sigma exp(\ ak\ +\ i\omega k\ )\ t \qquad\qquad (\ 8\ )$$

Here, k represents one of the frequency bands divided as desired.

When the pulse wave is observed for each frequency band k by the method according to the present disclosure for a desired period of time, the instantaneous logarithmic amplitude term ak and the instantaneous frequency term $\omega k$ are generally distributed like the Gaussian distribution when the physiological state is in a steady state. By observing the mean value and variance of the distribution, it is possible to comprehensively describe the physiological indices of short cyclic fluctuations of brain waves such as $\delta$ wave, $\theta$ wave, $\alpha$ wave, $\beta$ wave, and $\gamma$ wave from very long cyclic fluctuations such as diurnal fluctuation and seasonal fluctuation.

In the above example, the program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line. The computer includes various semiconductor devices such as a CPU, an MPU, an ECU, an FPGA and an ASIC.

The present disclosure is not limited to the embodiment described above, but only by the following claims.

## Claims

**1.** An autonomic nerve index calculation system (1) for calculating an autonomic nerve index of a living body, the autonomic nerve index calculation system (1) comprising:

a pulse wave waveform generation unit (101) configured to generate pulse wave waveform data using at least one pulse wave signal of the living body;
a band-pass filter (103) configured to filter the generated pulse wave waveform data in at least one predetermined frequency band;
a complex number conversion unit (104) configured to convert the filtered pulse wave waveform data into a complex number by Hilbert transformation and calculate pulse wave complex waveform data of the at least one frequency band; and
an index calculation unit (105) configured to calculate the autonomic nerve index of the living body in the at least one frequency band based on the calculated pulse wave complex waveform data, wherein
the index calculation unit (105) is configured to:

calculate at least one distribution of an instantaneous amplitude and an instantaneous frequency at predetermined time intervals, wherein the instantaneous amplitude corresponds to an absolute value of an amplitude term of the pulse wave complex waveform data and the instantaneous frequency corresponds to a time differential value of a phase term of the pulse wave complex waveform data; and
calculate at least one of a distribution coefficient of the instantaneous amplitude based on a distribution of the instantaneous amplitude and a distribution coefficient of the instantaneous frequency based on a distribution of the instantaneous frequency as the autonomic nerve index.

2. An autonomic nerve index calculation system (1) for calculating an autonomic nerve index of a living body, the autonomic nerve index calculation system comprising:

a heartbeat interval waveform generation unit (102) configured to generate heartbeat interval waveform data using at least one pulse wave signal of the living body;
a band-pass filter (103) configured to filter the generated heartbeat interval waveform data in at least one predetermined frequency band;
a complex number conversion unit (104) configured to convert the filtered heartbeat interval waveform data into a complex number by Hilbert transformation and calculate heartbeat interval complex waveform data of the at least one frequency band; and
an index calculation unit (105) configured to calculate the autonomic nerve index of the living body in the at least one frequency band based on the calculated heartbeat interval complex waveform data, wherein the index calculation unit (105) is configured to:

calculate at least one distribution of an instantaneous amplitude and an instantaneous frequency at predetermined time intervals, wherein the instantaneous amplitude corresponds to an absolute value of an amplitude term of the heartbeat interval complex waveform data and the instantaneous frequency corresponds to a time differential value of a phase term of the heartbeat interval complex waveform data; and
calculate at least one of a distribution coefficient of the instantaneous amplitude based on a distribution of the instantaneous amplitude and a distribution coefficient of the instantaneous frequency based on a distribution of the instantaneous frequency as the autonomic nerve index.

3. An autonomic nerve index calculation system (1) for calculating an autonomic nerve index of a living body, the autonomic nerve index calculation system comprising:

a pulse wave waveform generation unit (101) configured to generate pulse wave waveform data using at least one pulse wave signal of the living body;
a heartbeat interval waveform generation unit (102) configured to generate heartbeat interval waveform data using at least one pulse wave signal of the living body;
a band-pass filter (103) configured to filter the generated pulse wave waveform data and the heartbeat interval waveform data in at least one predetermined frequency band;
a complex number conversion unit (104) configured to convert the filtered pulse wave data and the filtered heartbeat interval waveform data into a complex number by Hilbert transformation and calculate pulse wave complex waveform data and heartbeat interval complex waveform data of the at least one frequency band; and
an index calculation unit (105) configured to calculate the autonomic nerve index of the living body in the at least one frequency band based on the calculated pulse wave complex waveform data and the calculated heartbeat interval complex waveform data, wherein the index calculation unit (105) is configured to:

calculate a distribution of an instantaneous phase difference at predetermined time intervals, wherein the instantaneous phase difference is a difference between a phase term of the pulse wave complex waveform data and a phase term of the heartbeat interval complex waveform data; and
calculate a distribution coefficient based on the distribution of the instantaneous phase difference as the autonomic nerve index.

4. An autonomic nerve index calculation method for calculating an autonomic nerve index of a living body, the autonomic nerve index calculation method comprising:

generating pulse wave waveform data using at least one pulse wave signal of the living body (S101);
filtering the generated pulse wave waveform data in at least one predetermined frequency band (S102);

converting the filtered pulse wave waveform data into a complex number by Hilbert transformation and calculate pulse wave complex waveform data of the at least one frequency band (S103); and

calculating the autonomic nerve index of the living body in the at least one frequency band based on the calculated pulse wave complex waveform data (S107, S108), wherein

the calculating the autonomic nerve index includes:

calculating at least one distribution of an instantaneous amplitude and an instantaneous frequency at predetermined time intervals, wherein the instantaneous amplitude corresponds to an absolute value of an amplitude term of the pulse wave complex waveform data and the instantaneous frequency corresponds to a time differential value of a phase term of the pulse wave complex waveform data; and

calculating at least one of a distribution coefficient of the instantaneous amplitude based on a distribution of the instantaneous amplitude and a distribution coefficient of the instantaneous frequency based on a distribution of the instantaneous frequency as the autonomic nerve index.

5. An autonomic nerve index calculation method for calculating an autonomic nerve index of a living body, the autonomic nerve index calculation method comprising:

generating heartbeat interval waveform data using at least one pulse wave signal of the living body (S104);

filtering the generated heartbeat interval waveform data in at least one predetermined frequency band (S105);

converting the filtered heartbeat interval waveform data into a complex number by Hilbert transformation and calculate heartbeat interval complex waveform data of the at least one frequency band (S106); and

calculating the autonomic nerve index of the living body in the at least one frequency band based on the calculated heartbeat interval complex waveform data (S107, S108), wherein

the calculating the autonomic nerve index includes:

calculating at least one distribution of an instantaneous amplitude and an instantaneous frequency at predetermined time intervals, wherein the instantaneous amplitude corresponds to an absolute value of an amplitude term of the heartbeat interval complex waveform data and the instantaneous frequency corresponds to a time differential value of a phase term of the heartbeat interval complex waveform data; and

calculating at least one of a distribution coefficient of the instantaneous amplitude based on a distribution of the instantaneous amplitude and a distribution coefficient of the instantaneous frequency based on a distribution of the instantaneous frequency as the autonomic nerve index.

6. An autonomic nerve index calculation method for calculating an autonomic nerve index of a living body, the autonomic nerve index calculation method comprising:

generating pulse wave waveform data using at least one pulse wave signal of the living body (S101);

generating heartbeat interval waveform data using at least one pulse wave signal of the living body (S104);

filtering the generated pulse wave waveform data and the heartbeat interval waveform data in at least one predetermined frequency band (S102, S105);

converting the filtered pulse wave data and the filtered heartbeat interval waveform data into complex numbers by Hilbert transformation and calculate pulse wave complex waveform data and heartbeat interval complex waveform data of the at least one frequency band (S103, S106); and

calculating the autonomic nerve index of the living body in the at least one frequency band based on the calculated pulse wave complex waveform data and the calculated heartbeat interval complex waveform data (S107, S108), wherein

the calculating the autonomic nerve index includes:

calculating a distribution of an instantaneous phase difference at predetermined time intervals, wherein the instantaneous phase difference is a difference between a phase term of the pulse wave complex waveform data and a phase term of the heartbeat interval complex waveform data; and

calculating a distribution coefficient based on the distribution of the instantaneous phase difference as the autonomic nerve index.

7. An autonomic nerve index calculation program (100) for calculating an autonomic nerve index of a living body for causing an information processing apparatus (40) to execute:

generating pulse wave waveform data using at least one pulse wave signal of the living body (S101);

filtering the generated pulse wave waveform data in at least one predetermined frequency band (S102);
converting the filtered pulse wave waveform data into a complex number by Hilbert transformation and calculate pulse wave complex waveform data of the at least one frequency band (S103); and
calculating the autonomic nerve index of the living body in the at least one frequency band based on the calculated pulse wave complex waveform data (S107, S108), wherein
the calculating the autonomic nerve index includes:

calculating at least one distribution of an instantaneous amplitude and an instantaneous frequency at predetermined time intervals, wherein the instantaneous amplitude corresponds to an absolute value of an amplitude term of the pulse wave complex waveform data and the instantaneous frequency corresponds to a time differential value of a phase term of the pulse wave complex waveform data; and
calculating at least one of a distribution coefficient of the instantaneous amplitude based on a distribution of the instantaneous amplitude and a distribution coefficient of the instantaneous frequency based on a distribution of the instantaneous frequency as the autonomic nerve index.

8. An autonomic nerve index calculation program (100) for calculating an autonomic nerve index of a living body for causing an information processing apparatus (40) to execute:

generating heartbeat interval waveform data using at least one pulse wave signal of the living body (S104);
filtering the generated heartbeat interval waveform data in at least one predetermined frequency band (S105);
converting the filtered heartbeat interval waveform data into a complex number by Hilbert transformation and calculate heartbeat interval complex waveform data of the at least one frequency band (S106); and
calculating the autonomic nerve index of the living body in the at least one frequency band based on the calculated heartbeat interval complex waveform data (S107, S108), wherein
the calculating the autonomic nerve index includes:

calculating at least one distribution of an instantaneous amplitude and an instantaneous frequency at predetermined time intervals, wherein the instantaneous amplitude corresponds to an absolute value of an amplitude term of the heartbeat interval complex waveform data and the instantaneous frequency corresponds to a time differential value of a phase term of the heartbeat interval complex waveform data; and
calculating at least one of a distribution coefficient of the instantaneous amplitude based on a distribution of the instantaneous amplitude and a distribution coefficient of the instantaneous frequency based on a distribution of the instantaneous frequency as the autonomic nerve index.

9. An autonomic nerve index calculation program (100) for calculating an autonomic nerve index of a living body for causing an information processing apparatus (40) to execute:

generating pulse wave waveform data using at least one pulse wave signal of the living body (S101);
generating heartbeat interval waveform data using at least one pulse wave signal of the living body (S104);
filtering the generated pulse wave waveform data and the heartbeat interval waveform data in at least one predetermined frequency band (S102,S105);
converting the filtered pulse wave data and the filtered heartbeat interval waveform data into complex numbers by Hilbert transformation and calculate pulse wave complex waveform data and heartbeat interval complex waveform data of the at least one frequency band (S103,S106); and
calculating the autonomic nerve index of the living body in the at least one frequency band based on the calculated pulse wave complex waveform data and the calculated heartbeat interval complex waveform data (S107, S108), wherein
the calculating the autonomic nerve index includes:

calculating a distribution of an instantaneous phase difference at predetermined time intervals, wherein the instantaneous phase difference is a difference between a phase term of the pulse wave complex waveform data and a phase term of the heartbeat interval complex waveform data; and
calculating a distribution coefficient based on the distribution of the instantaneous phase difference as the autonomic nerve index.

**Patentansprüche**

1. System zur Berechnung des autonomen Nervenindexes (1) zum Berechnen eines autonomen Nervenindexes eines lebenden Körpers, wobei das System zur Berechnung des autonomen Nervenindexes (1) umfasst:

   eine Erzeugungseinheit für Pulswellen-Wellenformen (101), die dafür ausgelegt ist, Pulswellen-Wellenformdaten unter Verwendung wenigstens eines Pulswellensignals des lebenden Körpers zu erzeugen;
   ein Bandpassfilter (103), das dafür ausgelegt ist, die erzeugten Pulswellen-Wellenformdaten in wenigstens einem vorbestimmten Frequenzband zu filtern;
   eine Umwandlungseinheit für komplexe Zahlen (104), die dafür ausgelegt ist, die gefilterten Pulswellen-Wellenformdaten durch Hilbert-Transformation in eine komplexe Zahl umzuwandeln und komplexe Pulswellen-Wellenformdaten des wenigstens einen Frequenzbandes zu berechnen; und
   eine Indexberechnungseinheit (105), die dafür ausgelegt ist, den autonomen Nervenindex des lebenden Körpers in dem wenigstens einen Frequenzband basierend auf den berechneten komplexen Pulswellen-Wellenformdaten zu berechnen, wobei
   die Indexberechnungseinheit (105) ausgelegt ist zum:

   Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Pulswellen-Wellenformdaten entspricht und die Momentanfrequenz einem Zeitdifferenzwert eines Phasenterms der komplexen Pulswellen-Wellenformdaten entspricht; und
   Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

2. System zur Berechnung des autonomen Nervenindexes (1) zum Berechnen eines autonomen Nervenindexes eines lebenden Körpers, wobei das System zur Berechnung des autonomen Nervenindexes umfasst:

   eine Erzeugungseinheit für Herzschlagintervall-Wellenformen (102), die dafür ausgelegt ist, Herzschlagintervall-Wellenformdaten unter Verwendung wenigstens eines Pulswellensignals des lebenden Körpers zu erzeugen;
   ein Bandpassfilter (103), das dafür ausgelegt ist, die erzeugten Herzschlagintervall-Wellenformdaten in wenigstens einem vorbestimmten Frequenzband zu filtern;
   eine Umwandlungseinheit für komplexe Zahlen (104), die dafür ausgelegt ist, die gefilterten Herzschlagintervall-Wellenformdaten durch Hilbert-Transformation in eine komplexe Zahl umzuwandeln und komplexe Herzschlagintervall-Wellenformdaten des wenigstens einen Frequenzbandes zu berechnen; und
   eine Indexberechnungseinheit (105), die dafür ausgelegt ist, den autonomen Nervenindex des lebenden Körpers in dem wenigstens einen Frequenzband basierend auf den berechneten komplexen Herzschlagintervall-Wellenformdaten zu berechnen, wobei
   die Indexberechnungseinheit (105) ausgelegt ist zum:

   Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Herzschlagintervall-Wellenformdaten entspricht und die Momentanfrequenz einem Zeitdifferenzwert eines Phasenterms der komplexen Herzschlagintervall-Wellenformdaten entspricht; und
   Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

3. System zur Berechnung des autonomen Nervenindexes (1) zum Berechnen eines autonomen Nervenindexes eines lebenden Körpers, wobei das System zur Berechnung des autonomen Nervenindexes umfasst:

   eine Erzeugungseinheit für Pulswellen-Wellenformen (101), die dafür ausgelegt ist, Pulswellen-Wellenformdaten unter Verwendung wenigstens eines Pulswellensignals des lebenden Körpers zu erzeugen;
   eine Erzeugungseinheit für Herzschlagintervall-Wellenformen (102), die dafür ausgelegt ist, Herzschlagintervall-Wellenformdaten unter Verwendung wenigstens eines Pulswellensignals des lebenden Körpers zu erzeugen;
   ein Bandpassfilter (103), das dafür ausgelegt ist, die erzeugten Pulswellen-Wellenformdaten und die Herzschlagintervall-Wellenformdaten in wenigstens einem vorbestimmten Frequenzband zu filtern;
eine Umwandlungseinheit für komplexe Zahlen (104), die dafür ausgelegt ist, die gefilterten Pulswellen-Wellenformdaten und die gefilterten Herzschlagintervall-Wellenformdaten durch Hilbert-Transformation in eine komplexe Zahl umzuwandeln und
komplexe Pulswellen-Wellenformdaten und komplexe Herzschlagintervall-Wellenformdaten des wenigstens einen Frequenzbandes zu berechnen; und
eine Indexberechnungseinheit (105), die dafür ausgelegt ist, den autonomen Nervenindex des lebenden Körpers in dem wenigstens einen Frequenzband basierend auf den berechneten komplexen Pulswellen-Wellenformdaten und
den berechneten komplexen Herzschlagintervall-Wellenformdaten zu berechnen, wobei
die Indexberechnungseinheit (105) ausgelegt ist zum:

Berechnen einer Verteilung einer momentanen Phasendifferenz in vorbestimmten Zeitintervallen, wobei die momentane Phasendifferenz eine Differenz zwischen einem Phasenterm der komplexen Pulswellen-Wellenformdaten und einem Phasenterm der komplexen Herzschlagintervall-Wellenformdaten ist; und
Berechnen eines Verteilungskoeffizienten basierend auf der Verteilung der momentanen Phasendifferenz als autonomen Nervenindex.

4. Verfahren zur Berechnung des autonomen Nervenindexes zum Berechnen eines autonomen Nervenindexes eines lebenden Körpers, wobei das Verfahren zur Berechnung des autonomen Nervenindexes umfasst:

Erzeugen von Pulswellen-Wellenformdaten unter Verwendung wenigstens eines Pulswellensignals des lebenden Körpers (S101);
Filtern der erzeugten Pulswellen-Wellenformdaten in wenigstens einem vorbestimmten Frequenzband (S102);
Umwandeln der gefilterten Pulswellen-Wellenformdaten durch Hilbert-Transformation in eine komplexe Zahl und Berechnen komplexer Pulswellen-Wellenformdaten des wenigstens einen Frequenzbandes (S103); und
Berechnen des autonomen Nervenindex des lebenden Körpers in dem wenigstens einen Frequenzband basierend auf den berechneten komplexen Pulswellen-Wellenformdaten (S107, S108), wobei
das Berechnen des autonomen Nervenindexes beinhaltet:

Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Pulswellen-Wellenformdaten entspricht und die Momentanfrequenz einem Zeitdifferenzwert eines Phasenterms der komplexen Pulswellen-Wellenformdaten entspricht; und
Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

5. Verfahren zur Berechnung des autonomen Nervenindexes zum Berechnen eines autonomen Nervenindexes eines lebenden Körpers, wobei das Verfahren zur Berechnung des autonomen Nervenindexes umfasst:

Erzeugen von Herzschlagintervall-Wellenformdaten unter Verwendung wenigstens eines Pulswellensignals des lebenden Körpers (S104);
Filtern der erzeugten Herzschlagsignal-Wellenformdaten in wenigstens einem vorbestimmten Frequenzband (S105);
Umwandeln der gefilterten Herzschlagintervall-Wellenformdaten durch Hilbert-Transformation in eine komplexe Zahl und Berechnen komplexer Herzschlagintervall-Wellenformdaten des wenigstens einen Frequenzbandes (S106); und
Berechnen des autonomen Nervenindex des lebenden Körpers in dem wenigstens einen Frequenzband basierend auf den berechneten komplexen Herzschlagintervall-Wellenformdaten (S107, S108), wobei
das Berechnen des autonomen Nervenindexes beinhaltet:

Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Herzschlagintervall-Wellenformdaten entspricht und die Momentanfrequenz einem Zeitdifferenzwert eines Phasenterms der komplexen Herzschlagintervall-Wellenformdaten entspricht; und
Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

6. Verfahren zur Berechnung des autonomen Nervenindexes zum Berechnen eines autonomen Nervenindexes eines lebenden Körpers, wobei das Verfahren zur Berechnung des autonomen Nervenindexes umfasst:

Erzeugen von Pulswellen-Wellenformdaten unter Verwendung wenigstens eines Pulswellensignals des lebenden Körpers (S101);
Erzeugen von Herzschlagintervall-Wellenformdaten unter Verwendung wenigstens eines Pulswellensignals des lebenden Körpers (S104);
Filtern der erzeugten Pulswellen-Wellenformdaten und der Herzschlagintervall-Wellenformdaten in wenigstens einem vorbestimmten Frequenzband (S102, S105);
Umwandeln der gefilterten Pulswellen-Wellenformdaten und der gefilterten Herzschlagintervall-Wellenformdaten durch Hilbert-Transformation in komplexe Zahlen und Berechnen komplexer Pulswellen-Wellenformdaten und komplexer Herzschlagintervall-Wellenformdaten des wenigstens einen Frequenzbandes (S103, S106); und
Berechnen des autonomen Nervenindexes des lebenden Körpers in dem wenigstens einen Frequenzband basierend auf den berechneten komplexen Pulswellen-Wellenformdaten und den berechneten komplexen Herzschlagintervall-Wellenformdaten (S107, S108), wobei das Berechnen des autonomen Nervenindexes beinhaltet:

Berechnen einer Verteilung einer momentanen Phasendifferenz in vorbestimmten Zeitintervallen, wobei die momentane Phasendifferenz eine Differenz zwischen einem Phasenterm der komplexen Pulswellen-Wellenformdaten und einem Phasenterm der komplexen Herzschlagintervall-Wellenformdaten ist; und
Berechnen eines Verteilungskoeffizienten basierend auf der Verteilung der momentanen Phasendifferenz als autonomen Nervenindex.

7. Programm zur Berechnung des autonomen Nervenindexes (100) zum Berechnen eines autonomen Nervenindexes eines lebenden Körpers, um eine Informationsverarbeitungsvorrichtung (40) zu veranlassen zum:

Erzeugen von Pulswellen-Wellenformdaten unter Verwendung wenigstens eines Pulswellensignals des lebenden Körpers (S101);
Filtern der erzeugten Pulswellen-Wellenformdaten in wenigstens einem vorbestimmten Frequenzband (S102);
Umwandeln der gefilterten Pulswellen-Wellenformdaten durch Hilbert-Transformation in eine komplexe Zahl und Berechnen komplexer Pulswellen-Wellenformdaten des wenigstens einen Frequenzbandes (S103); und
Berechnen des autonomen Nervenindex des lebenden Körpers in dem wenigstens einen Frequenzband basierend auf den berechneten komplexen Pulswellen-Wellenformdaten (S107, S108), wobei
das Berechnen des autonomen Nervenindexes beinhaltet:

Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Pulswellen-Wellenformdaten entspricht und die Momentanfrequenz einem Zeitdifferenzwert eines Phasenterms der komplexen Pulswellen-Wellenformdaten entspricht; und
Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

8. Programm zur Berechnung des autonomen Nervenindexes (100) zum Berechnen eines autonomen Nervenindexes eines lebenden Körpers, um eine Informationsverarbeitungsvorrichtung (40) zu veranlassen zum:

Erzeugen von Herzschlagintervall-Wellenformdaten unter Verwendung wenigstens eines Pulswellensignals des lebenden Körpers (S104);
Filtern der erzeugten Herzschlagsignal-Wellenformdaten in wenigstens einem vorbestimmten Frequenzband (S105);
Umwandeln der gefilterten Herzschlagintervall-Wellenformdaten durch Hilbert-Transformation in eine komplexe Zahl und Berechnen komplexer Herzschlagintervall-Wellenformdaten des wenigstens einen Frequenzbandes (S106); und
Berechnen des autonomen Nervenindex des lebenden Körpers in dem wenigstens einen Frequenzband basierend auf den berechneten komplexen Herzschlagintervall-Wellenformdaten (S107, S108), wobei
das Berechnen des autonomen Nervenindexes beinhaltet:

Berechnen wenigstens einer Verteilung einer Momentanamplitude und einer Momentanfrequenz in vorbestimmten Zeitintervallen, wobei die Momentanamplitude einem Absolutwert eines Amplitudenterms der komplexen Herzschlagintervall-Wellenformdaten entspricht und die Momentanfrequenz einem Zeitdifferenzwert eines Phasenterms der komplexen Herzschlagintervall-Wellenformdaten entspricht; und Berechnen wenigstens entweder eines Verteilungskoeffizienten der Momentanamplitude basierend auf einer Verteilung der Momentanamplitude und/oder eines Verteilungskoeffizienten der Momentanfrequenz basierend auf einer Verteilung der Momentanfrequenz als den autonomen Nervenindex.

9. Programm zur Berechnung des autonomen Nervenindexes (100) zum Berechnen eines autonomen Nervenindexes eines lebenden Körpers, um eine Informationsverarbeitungsvorrichtung (40) zu veranlassen zum:

Erzeugen von Pulswellen-Wellenformdaten unter Verwendung wenigstens eines Pulswellensignals des lebenden Körpers (S101);
Erzeugen von Herzschlagintervall-Wellenformdaten unter Verwendung wenigstens eines Pulswellensignals des lebenden Körpers (S104);
Filtern der erzeugten Pulswellen-Wellenformdaten und der Herzschlagintervall-Wellenformdaten in wenigstens einem vorbestimmten Frequenzband (S102, S105);
Umwandeln der gefilterten Pulswellen-Wellenformdaten und der gefilterten Herzschlagintervall-Wellenformdaten durch Hilbert-Transformation in komplexe Zahlen und Berechnen komplexer Pulswellen-Wellenformdaten und komplexer Herzschlagintervall-Wellenformdaten des wenigstens einen Frequenzbandes (S103, S106); und Berechnen des autonomen Nervenindexes des lebenden Körpers in dem wenigstens einen Frequenzband basierend auf den berechneten komplexen Pulswellen-Wellenformdaten und den berechneten komplexen Herzschlagintervall-Wellenformdaten (S107, S108), wobei das Berechnen des autonomen Nervenindexes beinhaltet:

Berechnen einer Verteilung einer momentanen Phasendifferenz in vorbestimmten Zeitintervallen, wobei die momentane Phasendifferenz eine Differenz zwischen einem Phasenterm der komplexen Pulswellen-Wellenformdaten und einem Phasenterm der komplexen Herzschlagintervall-Wellenformdaten ist; und
Berechnen eines Verteilungskoeffizienten basierend auf der Verteilung der momentanen Phasendifferenz als autonomen Nervenindex.

## Revendications

1. Système de calcul d'indice nerveux autonome (1) destiné à calculer un indice nerveux autonome d'un corps vivant, le système de calcul d'indice nerveux autonome (1) comprenant :

une unité de génération de formes d'onde d'ondes pulsées (101) configurée pour générer des données de forme d'onde d'onde pulsée en utilisant au moins un signal d'onde pulsée du corps vivant ;
un filtre passe-bande (103) configuré pour filtrer les données de forme d'onde d'onde pulsée générées dans au moins une bande de fréquence prédéterminée ;
une unité de conversion en nombre complexe (104) configurée pour convertir les données de forme d'onde d'onde pulsée filtrées en un nombre complexe par transformation de Hilbert et calculer des données de forme d'onde complexe d'onde pulsée de l'au moins une bande de fréquence ; et
une unité de calcul d'indice (105) configurée pour calculer l'indice nerveux autonome du corps vivant dans l'au moins une bande de fréquence sur la base des données de forme d'onde complexe d'onde pulsée calculées, dans lequel
l'unité de calcul d'indice (105) est configurée pour :

calculer au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'onde pulsée et la fréquence instantanée correspondant à une valeur différentielle temporelle d'un terme de phase des données de forme d'onde complexe d'onde pulsée ; et
calculer un coefficient de distribution de l'amplitude instantanée sur la base d'une distribution de l'amplitude instantanée et/ou un coefficient de distribution de la fréquence instantanée sur la base d'une distribution de la fréquence instantanée comme l'indice nerveux autonome.

**EP 4 005 468 B1**

2. Système de calcul d'indice nerveux autonome (1) destiné à calculer un indice nerveux autonome d'un corps vivant, le système de calcul d'indice nerveux autonome comprenant :

une unité de génération de formes d'onde d'intervalles de battement du coeur (102) configurée pour générer des données de forme d'onde d'intervalle de battement du coeur en utilisant au moins un signal d'onde pulsée du corps vivant ;
un filtre passe-bande (103) configuré pour filtrer les données de forme d'onde d'intervalle de battement du coeur générées dans au moins une bande de fréquence prédéterminée ;
une unité de conversion en nombre complexe (104) configurée pour convertir les données de forme d'onde d'intervalle de battement du coeur filtrées en un nombre complexe par transformation de Hilbert et calculer des données de forme d'onde complexe d'intervalle de battement du coeur de l'au moins une bande de fréquence ; et
une unité de calcul d'indice (105) configurée pour calculer l'indice nerveux autonome du corps vivant dans l'au moins une bande de fréquence sur la base des données de forme d'onde complexe d'intervalle de battement du coeur calculées, dans lequel
l'unité de calcul d'indice (105) est configurée pour :

calculer au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'intervalle de battement du coeur et la fréquence instantanée correspondant à une valeur différentielle temporelle d'un terme de phase des données de forme d'onde complexe d'intervalle de battement du coeur ; et
calculer un coefficient de distribution de l'amplitude instantanée sur la base d'une distribution de l'amplitude instantanée et/ou un coefficient de distribution de la fréquence instantanée sur la base d'une distribution de la fréquence instantanée comme l'indice nerveux autonome.

3. Système de calcul d'indice nerveux autonome (1) destiné à calculer un indice nerveux autonome d'un corps vivant, le système de calcul d'indice nerveux autonome comprenant :

une unité de génération de formes d'onde d'ondes pulsées (101) configurée pour générer des données de forme d'onde d'onde pulsée en utilisant au moins un signal d'onde pulsée du corps vivant ;
une unité de génération de formes d'onde d'intervalles de battement du coeur (102) configurée pour générer des données de forme d'onde d'intervalle de battement du coeur en utilisant au moins un signal d'onde pulsée du corps vivant ;
un filtre passe-bande (103) configuré pour filtrer les données de forme d'onde d'onde pulsée et les données de forme d'onde d'intervalle de battement du coeur générées dans au moins une bande de fréquence prédéterminée ;
une unité de conversion en nombre complexe (104) configurée pour convertir les données d'onde pulsée filtrées et les données de forme d'onde d'intervalle de battement du coeur filtrées en un nombre complexe par transformation de Hilbert et calculer des données de forme d'onde complexe d'onde pulsée et des données de forme d'onde complexe d'intervalle de battement du coeur de l'au moins une bande de fréquence ; et
une unité de calcul d'indice (105) configurée pour calculer l'indice nerveux autonome du corps vivant dans l'au moins une bande de fréquence sur la base des données de forme d'onde complexe d'onde pulsée calculées et des données de forme d'onde complexe d'intervalle de battement du coeur calculées, dans lequel
l'unité de calcul d'indice (105) est configurée pour :

calculer une distribution d'une différence de phase instantanée à intervalles de temps prédéterminés, la différence de phase instantanée étant une différence entre un terme de phase des données de forme d'onde complexe d'onde pulsée et un terme de phase des données de forme d'onde complexe d'intervalle de battement du coeur ; et
calculer un coefficient de distribution sur la base de la distribution de la différence de phase instantanée comme l'indice nerveux autonome.

4. Procédé de calcul d'indice nerveux autonome destiné à calculer un indice nerveux autonome d'un corps vivant, le procédé de calcul d'indice nerveux autonome comprenant :

la génération de données de forme d'onde d'onde pulsée au moyen d'au moins un signal d'onde pulsée du corps vivant (S101) ;
le filtrage des données de forme d'onde d'onde pulsée générées dans au moins une bande de fréquence

EP 4 005 468 B1

prédéterminée (S102) ;

la conversion des données de forme d'onde d'onde pulsée filtrées en un nombre complexe par transformation de Hilbert et le calcul de données de forme d'onde complexe d'onde pulsée de l'au moins une bande de fréquence (S103) ; et

le calcul de l'indice nerveux autonome du corps vivant dans l'au moins une bande de fréquence sur la base des données de forme d'onde complexe d'onde pulsée calculées (S107, S108), dans lequel

le calcul de l'indice nerveux autonome comporte :

le calcul d'au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'onde pulsée et la fréquence instantanée correspondant à une valeur différentielle temporelle d'un terme de phase des données de forme d'onde complexe d'onde pulsée ; et

le calcul d'un coefficient de distribution de l'amplitude instantanée sur la base d'une distribution de l'amplitude instantanée et/ou d'un coefficient de distribution de la fréquence instantanée sur la base d'une distribution de la fréquence instantanée comme l'indice nerveux autonome.

5. Procédé de calcul d'indice nerveux autonome destiné à calculer un indice nerveux autonome d'un corps vivant, le procédé de calcul d'indice nerveux autonome comprenant :

la génération de données de forme d'onde d'intervalle de battement du coeur au moyen d'au moins un signal d'onde pulsée du corps vivant (S104) ;

le filtrage des données de forme d'onde d'intervalle de battement du coeur générées dans au moins une bande de fréquence prédéterminée (S105) ;

la conversion des données de forme d'onde d'intervalle de battement du coeur filtrées en un nombre complexe par transformation de Hilbert et le calcul de données de forme d'onde complexe d'intervalle de battement du coeur de l'au moins une bande de fréquence (S106) ; et

le calcul de l'indice nerveux autonome du corps vivant dans l'au moins une bande de fréquence sur la base des données de forme d'onde complexe d'intervalle de battement du coeur calculées (S107, S108), dans lequel

le calcul de l'indice nerveux autonome comporte :

le calcul d'au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'intervalle de battement du coeur et la fréquence instantanée correspondant à une valeur différentielle temporelle d'un terme de phase des données de forme d'onde complexe d'intervalle de battement du coeur ; et

le calcul d'un coefficient de distribution de l'amplitude instantanée sur la base d'une distribution de l'amplitude instantanée et/ou d'un coefficient de distribution de la fréquence instantanée sur la base d'une distribution de la fréquence instantanée comme l'indice nerveux autonome.

6. Procédé de calcul d'indice nerveux autonome destiné à calculer un indice nerveux autonome d'un corps vivant, le procédé de calcul d'indice nerveux autonome comprenant :

la génération de données de forme d'onde d'onde pulsée au moyen d'au moins un signal d'onde pulsée du corps vivant (S101) ;

la génération de données de forme d'onde d'intervalle de battement du coeur au moyen d'au moins un signal d'onde pulsée du corps vivant (S104) ;

le filtrage des données de forme d'onde d'onde pulsée et des données de forme d'onde d'intervalle de battement du coeur générées dans au moins une bande de fréquence prédéterminée (S102, S105) ;

la conversion des données d'onde pulsée filtrées et des données de forme d'onde d'intervalle de battement du coeur filtrées en nombres complexes par transformation de Hilbert et le calcul de données de forme d'onde complexe d'onde pulsée et de données de forme d'onde complexe d'intervalle de battement du coeur de l'au moins une bande de fréquence (S103, S106) ; et

le calcul de l'indice nerveux autonome du corps vivant dans l'au moins une bande de fréquence sur la base des données de forme d'onde complexe d'onde pulsée calculées et des données de forme d'onde complexe d'intervalle de battement du coeur calculées (S107, S108), dans lequel

le calcul de l'indice nerveux autonome comporte :

le calcul d'une distribution d'une différence de phase instantanée à intervalles de temps prédéterminés, la différence de phase instantanée étant une différence entre un terme de phase des données de forme d'onde complexe d'onde pulsée et un terme de phase des données de forme d'onde complexe d'intervalle de battement du coeur ; et

le calcul d'un coefficient de distribution sur la base de la distribution de la différence de phase instantanée comme l'indice nerveux autonome.

**7.** Programme de calcul d'indice nerveux autonome (100) destiné à calculer un indice nerveux autonome d'un corps vivant servant à faire exécuter par un appareil de traitement d'informations (40) :

la génération de données de forme d'onde d'onde pulsée au moyen d'au moins un signal d'onde pulsée du corps vivant (S101) ;

le filtrage des données de forme d'onde d'onde pulsée générées dans au moins une bande de fréquence prédéterminée (S102) ;

la conversion des données de forme d'onde d'onde pulsée filtrées en un nombre complexe par transformation de Hilbert et le calcul de données de forme d'onde complexe d'onde pulsée de l'au moins une bande de fréquence (S103) ; et

le calcul de l'indice nerveux autonome du corps vivant dans l'au moins une bande de fréquence sur la base des données de forme d'onde complexe d'onde pulsée calculées (S107, S108), dans lequel

le calcul de l'indice nerveux autonome comporte :

le calcul d'au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'onde pulsée et la fréquence instantanée correspondant à une valeur différentielle temporelle d'un terme de phase des données de forme d'onde complexe d'onde pulsée ; et

le calcul d'un coefficient de distribution de l'amplitude instantanée sur la base d'une distribution de l'amplitude instantanée et/ou d'un coefficient de distribution de la fréquence instantanée sur la base d'une distribution de la fréquence instantanée comme l'indice nerveux autonome.

**8.** Programme de calcul d'indice nerveux autonome (100) destiné à calculer un indice nerveux autonome d'un corps vivant servant à faire exécuter par un appareil de traitement d'informations (40) :

la génération de données de forme d'onde d'intervalle de battement du coeur au moyen d'au moins un signal d'onde pulsée du corps vivant (S104) ;

le filtrage des données de forme d'onde d'intervalle de battement du coeur générées dans au moins une bande de fréquence prédéterminée (S105) ;

la conversion des données de forme d'onde d'intervalle de battement du coeur filtrées en un nombre complexe par transformation de Hilbert et le calcul de données de forme d'onde complexe d'intervalle de battement du coeur de l'au moins une bande de fréquence (S106) ; et

le calcul de l'indice nerveux autonome du corps vivant dans l'au moins une bande de fréquence sur la base des données de forme d'onde complexe d'intervalle de battement du coeur calculées (S107, S108), dans lequel

le calcul de l'indice nerveux autonome comporte :

le calcul d'au moins une distribution d'une amplitude instantanée et d'une fréquence instantanée à intervalles de temps prédéterminés, l'amplitude instantanée correspondant à une valeur absolue d'un terme d'amplitude des données de forme d'onde complexe d'intervalle de battement du coeur et la fréquence instantanée correspondant à une valeur différentielle temporelle d'un terme de phase des données de forme d'onde complexe d'intervalle de battement du coeur ; et

le calcul d'un coefficient de distribution de l'amplitude instantanée sur la base d'une distribution de l'amplitude instantanée et/ou d'un coefficient de distribution de la fréquence instantanée sur la base d'une distribution de la fréquence instantanée comme l'indice nerveux autonome.

**9.** Programme de calcul d'indice nerveux autonome (100) destiné à calculer un indice nerveux autonome d'un corps vivant servant à faire exécuter par un appareil de traitement d'informations (40) :

la génération de données de forme d'onde d'onde pulsée au moyen d'au moins un signal d'onde pulsée du corps vivant (S101) ;

la génération de données de forme d'onde d'intervalle de battement du coeur au moyen d'au moins un signal d'onde pulsée du corps vivant (S104) ;

le filtrage des données de forme d'onde d'onde pulsée et des données de forme d'onde d'intervalle de battement du coeur générées dans au moins une bande de fréquence prédéterminée (S102, S105) ;

la conversion des données d'onde pulsée filtrées et des données de forme d'onde d'intervalle de battement du coeur filtrées en nombres complexes par transformation de Hilbert et le calcul de données de forme d'onde complexe d'onde pulsée et de données de forme d'onde complexe d'intervalle de battement du coeur de l'au moins une bande de fréquence (S103, S106) ; et

le calcul de l'indice nerveux autonome du corps vivant dans l'au moins une bande de fréquence sur la base des données de forme d'onde complexe d'onde pulsée calculées et des données de forme d'onde complexe d'intervalle de battement du coeur calculées (S107, S108), dans lequel

le calcul de l'indice nerveux autonome comporte :

le calcul d'une distribution d'une différence de phase instantanée à intervalles de temps prédéterminés, la différence de phase instantanée étant une différence entre un terme de phase des données de forme d'onde complexe d'onde pulsée et un terme de phase des données de forme d'onde complexe d'intervalle de battement du coeur ; et

le calcul d'un coefficient de distribution sur la base de la distribution de la différence de phase instantanée comme l'indice nerveux autonome.

HEARTBEAT INTERVAL

PULSE WAVE

Fig. 1

HEARTBEAT
INTERVAL

SPLINE INTERPOLATION

TIME

Fig. 2

Fig. 3

Fig. 4

HEARTBEAT INTERVAL

----- PULSE WAVE

EQUATION (1)

PULSE WAVE ──── (HEARTBEAT INTERVAL + OUTPUT) ────▶ $\psi k(t) = \exp(ak\,(t) + i\omega k\,(t)\,t)$

HEARTBEAT INTERVAL ────▶ $\Psi k(t) = \exp(Ak\,(t) + i\Omega k\,(t)\,t)$

EQUATION (2)

EQUATION (3)

$\theta k\,(t) = \Omega k\,(t)\,t - \omega k\,(t)\,t$

$k = h, l, v, w, u, uu \cdots$
h ; HF[0.15:0.4]Hz
l ; LF[0.04:0.15]Hz
v ; VLF1[15:40]mHz
w ; VLF2[4:15]mHz
u ; ULF1[1.5:4]mHz
uu ; ULF2[0.4:1.5]mHz

Fig. 5

PULSE WAVE → BAND-PASS FILTER k → HILBERT TRANSFORMATION exp(ak(t)+iωk(t)t)

HEARTBEAT INTERVAL → BAND-PASS FILTER k → HILBERT TRANSFORMATION exp(Ak(t)+iΩk(t)t)

Outputs:

$a_k(t)$
$\omega_k(t)$
$\theta_k(t)$
$= \Omega_k(t)t - \omega_k(t)t$
$A_k(t)$
$\Omega_k(t)$

CAN BE APPROXIMATED TO GAUSSIAN DISTRIBUTION

$k = h, l, v, w\cdots$
h ; HF[0.15:0.4]Hz
l ; LF[0.04:0.15]Hz
v ; VLF1[15:40]mHz
w ; VLF2[4:15]mHz

Fig. 6

27

Fig. 7

Fig. 8

$\theta$ k DISTRIBUTION (VON MISES DISTRIBUTION)

Fig. 9A

θk DISTRIBUTION (VON MISES DISTRIBUTION)

Fig. 9B

PULSE INTERVAL WAVEFORM AMPLITUDE DISTRIBUTION (NORMAL DISTRIBUTION)

Fig. 10A

PULSE INTERVAL WAVEFORM AMPLITUDE DISTRIBUTION (NORMAL DISTRIBUTION)

Fig. 10B

ωk DISTRIBUTION (NORMAL DISTRIBUTION)

Fig. 11A

$\omega$k DISTRIBUTION (NORMAL DISTRIBUTION)

Fig. 11B

PULSE WAVE AMPLITUDE DISTRIBUTION (NORMAL DISTRIBUTION)

[0 : 60] SECONDS      HF: $\mu / \sigma = -4.36/0.46$

SPECTRUM IN HF

[0 : 60] SECONDS      LF: $\mu / \sigma = -3.64/0.59$

SPECTRUM IN LF

[0 : 600] SECONDS      VLF1: $\mu / \sigma = -2.82/0.73$

SPECTRUM IN VLF1

[0 : 600] SECONDS      VLF2: $\mu / \sigma = -3.62/0.88$

SPECTRUM IN VLF2

Fig. 12A

PULSE WAVE AMPLITUDE DISTRIBUTION (NORMAL DISTRIBUTION)

Fig. 12B

1／f

o2hb+hhb

$10^1$

$10^0$

$10^{-2}$　　　　$10^{-1}$

FREQUENCY （1／S）

Fig. 13

BRAIN STEM

PULSE PRESSURE

HEARTBEAT
$T_{i+1}=T_i+\Delta T_i$
$a_{i+1}=a_i+\Delta a_i$

BARORECEPTOR

PULSE PRESSURE (v)

SINOATRIAL NODE

ELASTICITY (C)

RESISTANCE (R) $\Rightarrow$　　　$\Leftarrow$ BLOOD FLOW (i) : INERTIA (L)

$$dv/dt = Rdi/dt+i/C+Ld^2i/dt^2$$

Fig. 14

Fig. 15

Fig. 16

EP 4 005 468 B1

PULSE WAVE WAVEFORM (k:H, L, V, W···)

$$\Psi_k(t) = \exp(a_k + i\omega_k t)$$
$$= \exp(a_k)\exp(i\omega_k t)$$
$$\omega_k = 2\pi/T_k$$

Fig. 17

EP 4 005 468 B1

TIME SERIES DATA IN BRAIN STEM (ASYNCHRONOUS/SYNCHRONOUS)

$T_r$    r: 0···4···9···14···18···24···29···33···38···42···47···51···55···60···64···69···74···78···83···88···93···98···103
$Th_i$    i: 0···1···2··· 3··· 4··· 5··· 6··· 7··· 8··· 9···10···11···12···13···14···15···16···17···18···19···20···21··· 22
$Tl_j$    j: 0···0···0··· 1··· 1··· 1··· 2··· 2··· 3··· 3··· 3··· 4··· 4··· 5··· 5··· 6··· 6··· 6··· 7··· 7··· 8··· 8··· 9
$Tv_n$    n: 0···0···0··· 0··· 0··· 1··· 1··· 1··· 1··· 1··· 1··· 1··· 2··· 2··· 2··· 2··· 3··· 3··· 3··· 3··· 3··· 4··· 4
$Tw_m$    m: 0···0···0··· 0··· 0··· 0··· 0··· 0··· 0··· 0··· 0··· 0··· 0··· 0··· 0··· 0··· 0··· 0··· 1··· 1··· 1··· 1

ORDINAL NUMBER
r : HEARTBEAT
i : RESPIRATION
j : BLOOD PRESSURE
n : AUTONOMIC NERVE 1
m : AUTONOMIC NERVE 2

Fig. 18

Fig. 19

Fig. 20

FREQUENCY (1/S)

Fig. 21

Fig. 22

Fig. 23

1

| 10 | 20 |
|---|---|
| PULSE WAVE SENSOR | AMPLIFIER |

30

A/D CONVERTER

| 40 | 50 |
|---|---|
| INFORMATION PROCESSING APPARATUS | DISPLAY APPARATUS |

60

STORAGE APPARATUS

Fig. 24

Fig. 25

START

| GENERATE PULSE WAVE WAVEFORM DATA | ～ S101 | | GENERATE HEARTBEAT INTERVAL WAVEFORM DATA | ～ S104 |

| FILTER PULSE WAVEFORM DATA | ～ S102 | | FILTER HEARTBEAT INTERVAL WAVEFORM DATA | ～ S105 |

| CONVERT FILTERED PULSE WAVEFORM DATA INTO COMPLEX NUMBER | ～ S103 | | CONVERT FILTERED HEARTBEAT INTERVAL WAVEFORM DATA INTO COMPLEX NUMBER | ～ S106 |

CALCULATE INSTANTANEOUS AMPLITUDE, CALCULATES THE INSTANTANEOUS AMPLITUDE OF THE PULSE WAVE COMPLEX WAVEFORM DATA, AND INSTANTANEOUS PHASE DIFFERENCE ～ S107

CALCULATE DISTRIBUTION COEFFICIENT OF INSTANTANEOUS AMPLITUDE, DISTRIBUTION COEFFICIENT OF INSTANTANEOUS FREQUENCY, AND DISTRIBUTION COEFFICIENT OF INSTANTANEOUS PHASE DIFFERENCE ～ S108

END

Fig. 26

Fig. 27

**REFERENCES CITED IN THE DESCRIPTION**

**Patent documents cited in the description**

- JP 5408751 B

- JP 2000296118 A

**Non-patent literature cited in the description**

- **W ZONG.** An Open-source Algorithm to Detect Onset of Arterial Blood Pressure Pulses. Harvard University-MIT Division of Health Sciences and Technology